# EUROPEAN PATENT APPLICATION

(11) **EP 1 908 849 A1**
(43) Date of publication of application: **09.04.2008**
(21) Application number: 06781776.7
(22) Date of filing: 27.07.2006
(51) Int. Cl.: C12Q 1/68, C12N 15/09

(54) **HYBRIDIZATION DETECTION METHOD**

(30) Priority: 27.07.2005 JP 2005217842; 20.06.2006 JP 2006170531
(71) Applicant: Sony Corporation, Minato-ku Tokyo 108-0075 (JP)
(72) Inventor: NAKAMURA, Ryota, c/o SONY CORPORATION, Tokyo 1410001 (JP); KISHII, Noriyuki, c/o SONY CORPORATION, Tokyo 1410001 (JP); ABE, Tomoteru, c/o SONY CORPORATION, Tokyo 1410001 (JP); YAMAMOTO, Takuro, c/o SONY CORPORATION, Tokyo 1410001 (JP); YAMASHITA, Shiko, c/o SONY CORPORATION, Tokyo 1410001 (JP); SEGAWA, Yuji, c/o SONY CORPORATION, Tokyo 1410001 (JP); OMORI, Shinji, c/o SONY CORPORATION, Tokyo 1410001 (JP)
(74) Representative: Mills, Julia
(86) International application number: PCT/JP2006/314865
(87) International publication number: WO 2007/013548

(57) **Abstract**

The present invention provides hybridization detection method capable of increasing an S/N ratio seen at hybridization detection time and, thus, improving the precision of detection of hybridization between a probe nucleic-acid chain and a target nucleic-acid chain. In accordance with the hybridization detection method, after the hybridization is advanced in a reaction space in which the hybridization takes place, a temperature increasing process is carried out in order to raise the temperature of the reaction space. In this way, the S/N ratio seen at hybridization detection time can be increased.

## Description

### [Technical Field]

The present invention relates to a technology for detecting hybridization. To put it in detail, the present invention relates to, among others, hybridization detection method for raising an S/N ratio seen at hybridization detection time by operating a temperature condition of a reaction space and other conditions after advancing hybridization of a probe nucleic-acid chain and a target nucleic-acid chain.

### [Background Art]

In recent years, efforts to put hybridization detection technology, which is used for detecting mainly hybridization making use of a DNA chip or a DNA microarray (hereinafter in this description referred to as "DNA chip"), to practical use have been making progress. A DNA chip is a chip obtained by integrating a number of DNA probes having a number of different kinds on a substrate and fixing the integrated DNA probes on the surface of the substrate. A process is carried out in order to detect hybridization of a probe nucleic-acid chain on the surface of the substrate of a DNA chip and a target nucleic-acid chain picked from a cell, a tissue or the like. By detecting such hybridization, a gene expression in the cell, the tissue or the like can be analyzed exhaustively.

In general, a process of detecting hybridization making use of a DNA chip is carried out in accordance with the following procedure. First of all, a sample nucleic acid is supplied to a reaction space in which a probe nucleic-acid chain is fixed. Then, hybridization between the probe nucleic-acid chain fixed in the reaction space and a target nucleic-acid chain in the sample nucleic acid is carried forward. Finally, an intercalater or the like is supplied to the reaction space and hybridization is detected by measuring the intensity of fluorescence generated by the intercalater.

References published before this description include Japanese Patent Laid-open No. 2003-300 and Japanese Patent Laid-open No. 2001-255328. The former reference discloses a base-sequence or nucleic-acid quantity determination method whereby the direction of the same sequence in a DNA chip is fixed in the vertical direction and a temperature gradient is provided in the vertical direction. On the other hand, the latter reference discloses hybridization-reaction detection method including the step of detecting hybridization reaction at every individual spot while raising the temperature of a biochip.

The probe nucleic-acid chain carries out not only hybridization in conjunction with a target nucleic-acid chain having a regular complementary chain, which is complementary to the probe nucleic-acid chain, but also hybridization (mis-hybridization) in conjunction with a nucleic acid having a homology with respect to only a portion of the base sequence of the probe nucleic-acid chain. Thus, the probe nucleic-acid chain may generate a mismatching double-strand nucleic acid in the reaction space as a result. A detected signal (for example, fluorescence) detected as the original signal of the mismatching double-strand nucleic acid becomes a noise (or a background noise). For this reason, there is a technological problem that the precision of the hybridization detection is not necessarily sufficient if the hybridization detection is carried out in order to quantitatively detect a target nucleic-acid chain.

In addition, in a process to detect hybridization by making use of an intercalater to be bound in a peculiar way to a double-strand nucleic acid obtained as a result of the hybridization, in some cases, the intercalater may be bound to a self-loop structure possibly created on a portion of a single-strand nucleic acid existing in the reaction space obtained as a result of a mis-hybridization or the intercalater may be absorbed in a non-peculiar way by the single-strand nucleic acid. Accordingly, there is another technological problem that noise fluorescence (or background-noise fluorescence) is generated at hybridization time.

It is thus a main object of the present invention to improve the precision of the hybridization detection by raising an S/N ratio seen at hybridization detection time.

### [Disclosure of Invention]

As a result of research carried out earnestly by inventors of the present invention in order to raise the S/N ratio in a process to detect hybridization, the inventors have completed a novel invention described below as an invention for effectively improving the S/N ratio by carrying out a process to properly adjust the temperature of a reaction space after advancing the hybridization in the reaction space. The S/N ratio is defined as a ratio of a normal detected signal (S) useful for the process to detect the hybridization to a noise signal (or a background-noise signal, N), which is not useful for the process to detect the hybridization.

First of all, the inventors have innovated hybridization detection method whereby, after hybridization between a probe nucleic-acid chain and a target nucleic-acid chain is advanced, a temperature increasing process is carried out in order to raise the temperature of a reaction space in which the hybridization is taking place to a value higher than a temperature determined in advance. By raising the temperature in this way, the S/N ratio seen at hybridization detection time can be improved. It is to be noted that the "temperature increasing process" is a process to shift the present temperature condition to a higher-temperature condition.

The method and/or means for carrying out the temperature increasing process are implemented by adoption of, for example, a technique of actually increasing the temperature of the reaction space in which the hybridization is taking place or a technique of making use of a higher-temperature solution (a technique of replacing the solution of the reaction space with a higher-temperature solution). It is to be noted, however, the method and/or means of the temperature changing process are by no means limited to these techniques. In addition, in the temperature increasing process carried out after the hybridization is advanced, the temperature of the reaction space is set at a higher temperature in order to disassociate an unnecessary target single-strand nucleic-acid chain from only a selected mismatching double-strand nucleic acid obtained as a result of a mis-hybridization. The higher temperature is a temperature, which is higher than the melting temperature (Tm) of the mismatching double-strand nucleic acid but lower than the Tm of a fully matching double-strand nucleic acid obtained as a result of a normal hybridization.

In this present invention, if a probe nucleic-acid chain having a base sequence varying from spot to spot (as a position included within the range of the reaction space as a position on which a drop of sample solution falls) in the reaction space is fixed in the reaction space, the temperature increasing processes each designed for a spot can be carried out independently of each other on the basis of the melting temperatures of the probe nucleic-acid chain and the target nucleic-acid chain.

As an alternative, in the temperature increasing process, the difference between a reaction equilibrium constant of the hybridization and a reaction equilibrium constant of the mis-hybridization can be raised in order to better disassociate an unnecessary target single-strand nucleic-acid chain from a selected mismatching double-strand nucleic acid obtained as a result of the mis-hybridization. In addition, in the temperature increasing process, a technique of in an instant raising the temperature of the reaction space to a temperature determined in advance is also useful means. This is because, by in an instant raising the temperature of the reaction space to a temperature determined in advance, it is possible to better disassociate an unnecessary target single-strand nucleic-acid chain from a selected mismatching double-strand nucleic acid obtained as a result of the mis-hybridization.

Next, hybridization detection methods provided by the present invention include a first typical hybridization detection method and, more desirably, a second typical hybridization detection method. The first typical hybridization detection method is a method of letting a target nucleic-acid chain marked in advance with a fluorescence chromatophore experience hybridization in conjunction with a probe nucleic-acid chain in order to detect fluorescence originated by the fluorescence chromatophore. On the other hand, the second typical hybridization detection method is a method of detecting hybridization between the target nucleic-acid chain and the probe nucleic-acid chain or to be bound to the double-strand nucleic acid by making use of an intercalater to be absorbed by a double-strand nucleic acid obtained as a result of the hybridization. It is to be noted, however, that the hybridization detection methods provided by the present invention are by no means limited to these first and second typical hybridization detection methods.

In accordance with the first hybridization detection method, the temperature increasing process is carried out properly in order to disassociate an unnecessary target single-strand nucleic-acid chain from a selected mismatching double-strand nucleic acid and, then, an unnecessary target single-strand nucleic-acid chain is removed from the reaction space in a cleaning process in order to reduce the intensity of noise fluorescence. In accordance with the second typical hybridization detection method making use of an intercalater, on the other hand, the temperature increasing process is carried out properly in order to disassociate an unnecessary target single-strand nucleic-acid chain from a selected mismatching double-strand nucleic acid existing in the reaction space. In this way, it is possible to reduce the intensity of noise fluorescence generated by the intercalater absorbed by the mismatching double-strand nucleic acid obtained as a result of a mis-hybridization or bound to the mismatching double-strand nucleic acid.

In addition, in order to implement the second typical hybridization detection method making use of an intercalater, in particular, it is desirable to carry out at least: a nucleic-acid chain supplying process of supplying a target nucleic-acid chain to a reaction space in which a probe nucleic-acid chain has been held or fixed; a first temperature increasing process of increasing the temperature of the reaction space; an intercalater supplying process of supplying an intercalater to the reaction space; a second temperature increasing process of re-increasing the temperature of the reaction space; and hybridization detection process of detecting hybridization by making use of the intercalater.

In particular, the second temperature increasing process is carried out by executing one or both of the following processes: (1) a process of disassociating the intercalater absorbed by a single-strand nucleic acid existing in the reaction space as a result of a mis-hybridization from the single-strand nucleic acid; and (2) a process of decomposing a self-loop structure created in the single-strand nucleic acid existing in the reaction space.

In addition, in accordance with the present invention, it is possible to give a result desirable for contribution to the improvement of the S/N ratio by carrying out a process of applying an electric field to the reaction space, which serves as the space of hybridization, at a proper stage.

For example, the process of applying an electric field to the reaction space is carried out at the stage of advancing hybridization. By virtue of an electrodynamical effect (for example, a dielectrophoresis or electrophresis) obtained as a result of applying an electric field to the reaction space, the target nucleic-acid chain is moved to a position close to the probe nucleic-acid chain. Thus, the hybridization can be promoted.

The application of an electric field to the reaction space can also be used to promote a process to disassociate an unnecessary target single-strand nucleic acid from a selected mismatching double-strand nucleic acid. For example, by applying a proper electric field to the reaction space in the process of increasing the temperature of the reaction space or in a process before or after execution of the temperature increasing process, a mismatching double-strand nucleic acid existing in the reaction space can be selected as a double-strand nucleic acid from which an unnecessary target single-strand nucleic acid is to be disassociated. As a result, a fully matching double-strand nucleic acid (a double-strand nucleic acid resulting from a normal hybridization as a double-strand nucleic acid including no mismatching single-strand nucleic acids) can be selectively sustained.

In addition, by applying a proper electric field to the reaction space, it is possible to increase the nucleic-acid chain concentration in the vicinity of a probe nucleic-acid chain existing in the reaction space. Thus, when a mismatching double-strand nucleic acid is selected as a mismatching double-strand nucleic acid from which an unnecessary target single-strand nucleic acid is to be disassociated by carrying out the process of increasing the temperature of the reaction space, due to a concentration gradient increased by the process of applying a proper electric field to the reaction space, the disassociated target single-strand nucleic acid more easily spreads to an area farther from the vicinity of the probe nucleic-acid chain in the solution. As a result, the process of applying a proper electric field to the reaction space can be expected to have an effect that a mis-hybridization hardly occurs as a reaction to reform the mismatching double-strand nucleic acid.

In addition, in the present invention, the probe nucleic-acid chain used for detection of hybridization is fixed in the reaction space and a process to clean the solution in the reaction space can be carried out after execution of the process of increasing the temperature of the reaction space on the basis of a condition set to require that a fully matching double-strand nucleic acid obtained as a result of hybridization with the probe nucleic-acid chain shall remain in the reaction space.

The process to clean the solution in the reaction space is a process useful for removing a noise generating target single-strand nuclei acid, which has been disassociated by the temperature increasing process and/or the field applying process from a mismatching double-strand nucleic acid, in advance prior to the hybridization detection stage.

Furthermore, the process to clean the solution in the reaction space can be carried out after application of an electric field to the reaction space already subjected to the temperature increasing process. In addition, the process to apply an electric field to the reaction space and the process to clean the solution in the reaction space can be carried out repeatedly several times. Thus, the process to apply an electric field to the reaction space causes a selective disassociation process to be carried out only on a mismatching double-strand nucleic acid in the reaction space including both the mismatching double-strand nucleic acid and a fully matching double-strand nucleic acid whereas the process to clean the solution in the reaction space removes a target nucleic-acid chain disassociated from the mismatching double-strand nucleic acid in the selective disassociation process. By carrying out the process to apply an electric field to the reaction space and the process to clean the solution in the reaction space repeatedly several times, the selective disassociation of a mismatching double-strand nucleic acid can be further advanced. As a result, a fully matching double-strand nucleic acid and a (fixed) probe nucleic-acid chain remain in the reaction space. The probe nucleic-acid chain remaining in the reaction space is a complementary chain, which is one of single-strand nucleic-acid chains composing a mismatching double-strand nucleic acid.

Technical terms used in the description of the present invention are explained as follows.

The technical term "reaction space" means a space that can be used for carrying out hybridization reaction.

The technical term "probe nucleic-acid chain" means a nucleic-acid chain functioning as a search needle (or a detector) for looking for a target nucleic-acid chain to be subjected to hybridization in conjunction with the probe nucleic-acid chain. The probe nucleic-acid chain exists in the reaction space in a state of being able to move freely or is placed in the reaction space by fixing one of the ends of the probe nucleic-acid chain on the surface of a material such as a substrate, a bead or a fiber. Representative probe nucleic-acid chains include the oligonucleotide or polynucleotide of a DNA probe.

The technical term "target nucleic-acid chain" means a nucleic-acid chain having a characteristic perfectly complementary to the base sequence of a probe nucleic-acid chain. In a process to detect hybridization, a target nucleic-acid chain is dropped or supplied to the reaction space. It is to be noted that the technical term "sample nucleic acid" is a nucleic acid dropped or supplied to the reaction space. Thus, a sample nucleic acid may or may not include a target nucleic-acid chain.

The technical term "nucleic acid" means a polymer (or a nucleotide chain) of a phosphorous acid ester of a nucleoside obtained as a result of binding a purine or pyrimidine base to a sugar in a glycoside junction process. The meaning of the technical term "nucleic acid" covers a wide range of acids including a DNA (the total length or its fragment) obtained as a result polymerization of an oligonucleotide, a polynucleotide, a purine nucleotide and a pyrimidine nucleotide, a cDNA (or a c probe DNA) obtained as a result of a reverse transcription and an RNA.

The technical term "hybridization" means a complementary-junction formation reaction of nucleic chains (or nucleotide chains). The hybridization includes DNA-DNA, DNA-RNA and RNA-RNA complementary junction formation reactions. The technical term "mis-hybridization" means a reaction forming a double-strand nucleic acid in a state of having mismatching members obtained as a result of a process of creating a complementary junction between mismatching bases (or between incompatible bases).

The technical term "fully matching double-strand nucleic acid" means a double-strand nucleic acid formed by a (normal) hybridization. The technical term "mismatching double-strand nucleic acid" means a double-strand nucleic acid formed by a mis-hybridization as a double-strand nucleic acid having mismatching members.

The technical term "melting temperature (Tm)" means a temperature in which a complementary junction (or hybridization) between nucleic acids is dissolved.

The technical term "self-loop structure" means a protruding structural portion obtained as a result of creating a loop shape at a part of a nucleic-acid chain composing a single-strand or double-strand chain. A self-loop structure may or may not have a portion forming a pair of complementary bases.

The technical term "S/N ratio" means a ratio of a normal detected signal (S) useful for a process to detect hybridization to a noise signal (or a background-noise signal, N), which is not useful for the process to detect hybridization.

The technical term "noise" means a detected signal representing a background noise in a process to detect hybridization. In particular, the noise is the fluorescence intensity of a detected signal representing a background noise. It is to be noted that, in this present invention, for example, an intercalater bound in a peculiar way to a complementary chain portion (or a fully matching double-strand nucleic acid) obtained as a result of a (normal) hybridization generates normal fluorescence. In addition, an intercalater bound in a non-peculiar way to a nucleic acid member other than the complementary chain portion generates fluorescence referred to as noise fluorescence.

The technical term "intercalater" means a substance bound in a peculiar way to or absorbed in a peculiar way by complementary junction members of a fully matching double-strand nucleic acid as a substance having a property of generating fluorescence. An intercalater is a substance used for detecting hybridization. Examples of the intercalater are POPO-1, TOTO-3 and SYBR (a registered trademark) Green I. It is to be noted that, in some cases, the intercalater is undesirably bound to or absorbed by members other than the normal (fully matching) complementary chain portion in a non-peculiar way. In addition, in some other cases, also in a non-peculiar way, the intercalater is undesirably bound to or absorbed by a complementary chain portion or self-loop structure portion of a mismatching double-strand nucleic acid, a single-strand nucleic acid put in a state of being free to move or fixed or an excessive single-strand member of a double-strand nucleic acid to mention a few. If the intercalater is bound in a non-peculiar way to or absorbed in a non-peculiar way by such members, the intercalater may generate noise fluorescence.

By virtue of the present invention, it is possible to increase the S/N ratio and improve the precision of the hybridization detection in a process to detect hybridization.

### [Brief Description of Drawings]

[Fig. 1] Fig. 1 is a diagram showing a model of a typical basic principle of hybridization detection adopting hybridization detection method provided by the present invention.

[Fig. 2] Fig. 2 is a diagram showing a model of another typical basic principle of hybridization detection adopting hybridization detection method provided by the present invention.

[Fig. 3] Fig. 3 is a flow diagram showing the process flow of a first typical hybridization detection method provided by the present invention.

[Fig. 4] Fig. 4 is a flow diagram showing the process flow of a second typical hybridization detection method provided by the present invention.

[Fig. 5] Fig. 5 is a conceptual diagram showing an outline of an additional process that can be used in hybridization process.

[Fig. 6] Fig. 6 is a flow diagram showing the process flow of hybridization detection method provided by the present invention.

[Fig. 7] Fig. 7 is a histogram representing fluorescence intensities obtained as a result of high-temperature processing carried out on fully-matching and mismatching nucleic acids.

[Fig. 8] Fig. 8 is histograms each representing fluorescence intensities obtained as a result of high-temperature processing carried out on fully-matching and mismatching nucleic acids.

[Fig. 9] Fig. 9 is a diagram showing temperature characteristics of the fluorescence intensity.

[Fig. 10] Fig. 10 is a diagram showing S/N ratios of the fluorescence intensities shown in Fig. 9.

[Fig. 11] Fig. 11 is a diagram showing a simulation model.

[Fig. 12] Fig. 12 is a diagram showing results of a simulation for a process of executing hybridizations for a fully matching nucleic acid and a mismatching nucleic acid at a temperature of 65 and then raising the temperature to 80.

[Fig. 13] Fig. 13 is diagrams showing simulation results representing relations between hybridization quantities and temperatures of a reaction space as well as relations between concentrations of a target nucleic acid existing in a diffusion layer and temperatures of a reaction space for hybridizations making use of fully matching and mismatching nucleic acids.

[Fig. 14] Fig. 14 is a diagram showing models of a behavior obtained from a simulation as the behavior of hybridization reaction.

[Fig. 15] Fig. 15 is a diagram (diagram substitute graph) showing results of experiments according to a fifth embodiment.

[Fig. 16] Fig. 16 is a diagram showing a model of the structure of a substrate used in an experiment of applying an electric field.

[Fig. 17] Fig. 17 is a diagram (diagram substitute graph) representing results of experiments according to a sixth embodiment.

[Fig. 18] Fig. 18 is a diagram (diagram substitute graph) showing results of experiments according to a seventh embodiment.

### [Best Modes for Carrying out the Invention]

Embodiments each implementing a method for detecting hybridization in accordance with the present invention are explained by referring to diagrams as follows.

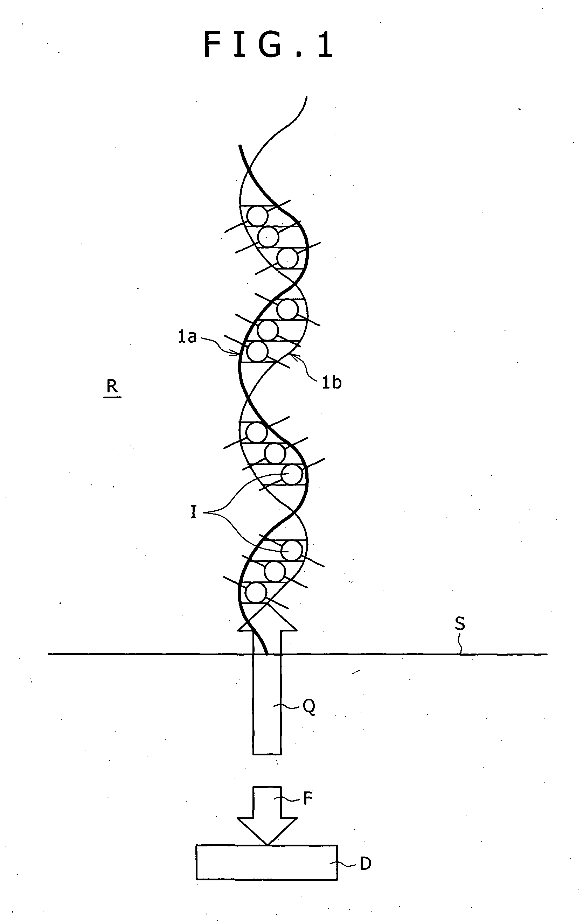

To begin with, Fig. 1 is given as a diagram showing a model of a typical basic principle of hybridization detection adopting hybridization detection method provided by the present invention.

Fig. 1 shows a state in which hybridization is advanced to create a complementary chain (double-strand nucleic acid). The hybridization is advanced as hybridization between a probe nucleic-acid chain 1a in a state of having its one end fixed on a surface (for example, a substrate, a beads and a fiber surface) denoted by notation S and a target nucleic-acid chain 1b introduced to a reaction space R in which the probe nucleic-acid chain 1a exists.

It is to be noted that, in this present invention, the end forming a part of the probe nucleic-acid chain 1a as the end fixed on the surface S can be either of ends 3' and 5' (not shown in the figure in particular). In addition, if a process to clean a solution as described later is not carried out, the state of the probe nucleic-acid chain 1a does not have to be the state of having its the end of the probe nucleic-acid chain 1a fixed on the surface S. For example, the probe nucleic-acid chain 1a can also be put in a state of freely moving in the reaction space R.

Notation I shown in Fig. 1 denotes an intercalater supplied in a non-peculiar way to be bound to the aforementioned complementary portion, which is obtained as a result of the hybridization. In a typical process of detecting the intensity of the excitation fluorescence F, a fluorescence excitation light Q generated by an external source (for example, a laser light beam having a wave length determined in advance) not shown in the figure as a light beam for exciting the generation of the excitation fluorescence F from the intercalater I is converged and radiated to the inside of the reaction space R and the intensity (fluorescence quantity) of the excitation fluorescence F generated by the intercalater I is measured by light detection means D such as the generally known photo detector or the like. The intensity of the excitation fluorescence F is measured in order to detect the hybridization.

In this case, the intercalater I is also undesirably bound to portions such as an excessive single-strand portion and/or a nucleic-acid chain portion creating a self-loop structure in a non-peculiar way, inadvertently generating a fluorescence (a noise fluorescence). The excessive single-strand portion is a portion of the single-strand nucleic acid in a state of freely moving in the reaction space R or a portion of a one-side nucleic acid (for example, the target nucleic-acid chain 1b, which is longer than the probe nucleic-acid chain 1a) forming the complementary chain. It is thus the main object of the present invention to reduce the quantity of the noise fluorescence.

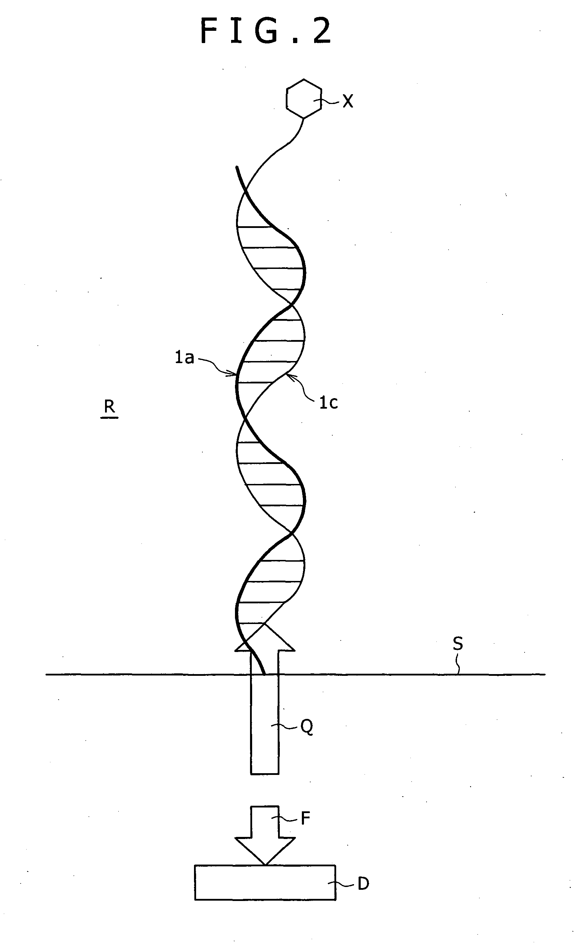

Next, Fig. 2 is given as a diagram showing a model of another typical basic principle of hybridization detection adopting hybridization detection method provided by the present invention.

Fig. 2 shows a state in which hybridization is advanced to create a complementary chain (which is a double-strand nucleic acid). The hybridization is advanced as hybridization between a probe nucleic-acid chain 1a in a state of having its one end fixed on a surface denoted by notation S (for example, a substrate, a beads and a fiber surface) and a target nucleic-acid chain 1c introduced to a reaction space R in which the probe nucleic-acid chain 1a exists.

On the target nucleic-acid chain 1c, a chromatophore X determined in advance is put as a label. A fluorescence excitation light Q generated by an external source (for example, a laser light beam having a wave length determined in advance) not shown in the figure as a light beam for exciting the generation of an excitation fluorescence F from the chromatophore X is converged and radiated to the inside of the reaction space R. A fluorescence quantity representing the intensity (fluorescence quantity) of the excitation fluorescence F generated by the chromatophore X is measured by light detection means D such as the generally known photo detector or the like. The intensity of the excitation fluorescence F is measured in order to detect hybridization.

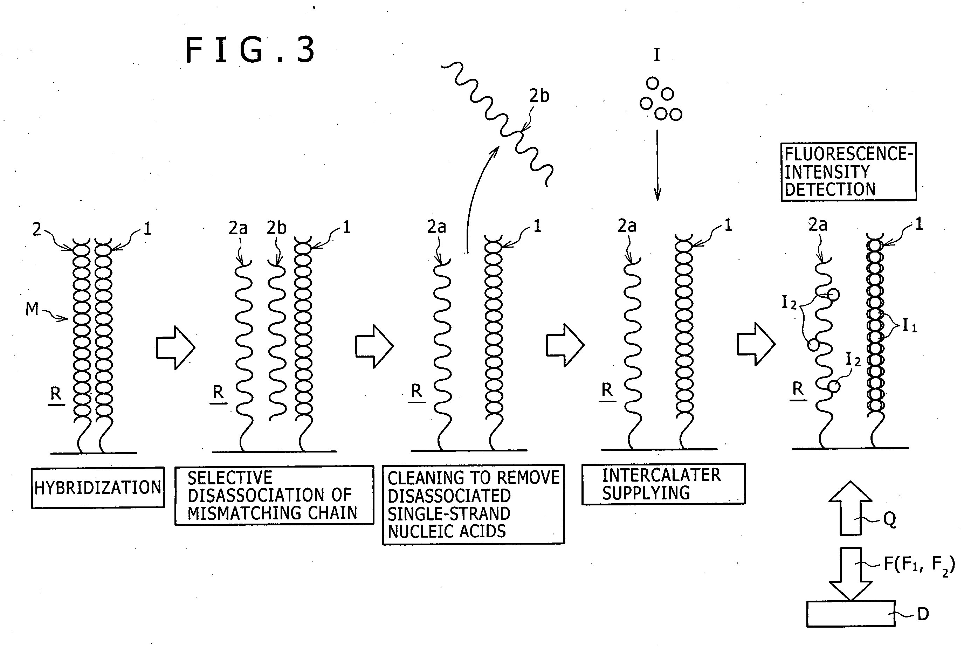

By referring to Figs. 3 to 7, the following description explains some preferred examples of processes related to the method for detecting hybridization in accordance with the present invention. It is to be noted that the following typical processes are each a representative process in which hybridization is detected by making use of an intercalater.

First of all, a first hybridization detection method shown in Fig. 3 includes main processes, which are sequentially listed as follows: hybridization; a selective disassociation of a mismatching double-strand nucleic acid (abbreviated to a mismatching chain); cleaning to remove disassociated single-strand nucleic acid; an intercalater supplying; and a fluorescence detection (hybridization detection). The processes listed above are explained sequentially as follows.

### (Hybridization Process)

A sample nucleic acid supplied to the reaction space R is assumed to include a target nucleic-acid chain having a base sequence portion perfectly complementary to the base sequence of a probe nucleic-acid chain and a target nucleic-acid chain having a base sequence portion partially incomplementary to the base sequence of the probe nucleic-acid chain. Thus, a normal hybridization creates a fully matching double-strand nucleic acid 1 from the target nucleic-acid chain having a perfectly complementary base sequence portion and a mismatching double-strand nucleic acid 2 having a mismatched member M from the target nucleic-acid chain having a partially incomplementary base sequence portion (referred to Fig. 3). It is to be noted that conditions for the hybridization can be set with a high degree of freedom in accordance with the objective of the hybridization detection method.

### (Selective Disassociation Process for a Mismatching Chain)

After the hybridization process described above is performed, a selective disassociation process is carried out to disassociate an unnecessary target single-strand nucleic acid from the deliberately selected mismatching double-strand nucleic acid 2. That is to say, the selective disassociation process is carried out not on the fully matching double-strand nucleic acid 1 but on the mismatching double-strand nucleic acid 2 only.

The selective disassociation process of disassociating the mismatching double-strand nucleic acid 2 is carried out by adoption of, for example, a technique of increasing the temperature of the environment of a solution in the reaction space R and the fully matching double-strand nucleic acid 1 is sustained in the reaction space R as it is. In Fig. 3, notation 2a denotes a probe nucleic-acid chain (single-strand chain) remaining in the mismatching double-strand nucleic acid 2 whereas notation 2b denotes a single-strand nucleic acid (hereinafter referred to as disassociated single-strand nucleic acid) disassociated from the probe single-strand nucleic-acid chain 2a.

It is to be noted that, if a probe nucleic-acid chain having a base sequence varying from spot to spot in the reaction space R is fixed in the reaction space R, temperature increasing processes each provided for a spot can be carried out independently of each other on the basis of the Tm of the probe nucleic-acid chain and the target nucleic-acid chain. By execution of independent optimum temperature control for every spot, variations of the hybridization quantity from spot to spot can be suppressed. As typical means for locally heating each of the spots, it is possible to adopt a Peltier technique, a Pt technique, an IR-laser technique, an induction (DC or AC) heating technique or a heat generating resistor technique (such as a TFT).

In the temperature increasing process, the difference between a reaction equilibrium constant of the normal hybridization and a reaction equilibrium constant of the mis-hybridization can be raised in order to better disassociate an double-strand nucleic acid 2 obtained as a result of the hybridization.

In addition, in this process, an electric field can be applied to the reaction space R in order to further advance the process of the mismatching double-strand nucleic acid 2. To put it more concretely, the selective disassociation process to disassociate the target single-strand nucleic acid 2b from the selected mismatching double-strand nucleic acid 2 is advanced and an electrical condition capable of providing the reaction space R with an electrodynamical effect capable of sustaining the double-strand state of the fully-matching double-strand nucleic acid 1 is selected to be followed by application of an electric field to the reaction space R.

The process to apply an electric field to the reaction space R can be carried out by combining the electric-field applying process with the process to increase the temperature. For example, the process to apply an electric field to the reaction space R and the process to increase the temperature of the reaction space R can be carried out in any order. As an alternative, the process to apply an electric field to the reaction space R and the process to increase the temperature of the reaction space R are carried out concurrently. By applying an electric field to the reaction space R, a nucleic-acid chain can be further concentrated in an area included in the reaction space R as an area in which the probe nucleic-acid chain exists, making the nucleic acid concentration non-uniform. Thus, when a mismatching double-strand nucleic acid 2 is to be disassociated by carrying out the process of increasing the temperature, due to a concentration gradient, the disassociated single-strand nucleic acid more easily spreads to an area farther from the vicinity of the probe nucleic-acid chain in the solution. As a result, it is possible to lower the probability that the mismatching double-strand nucleic acid 2 is recreated.

It is to be noted that, from the standpoint of the necessity to very effectively prevent the ion solution from undergoing an electrolysis on a boundary surface of an electrode facing the reaction space R (not shown) and other points of view, it is desirable to apply an AC electric field to the reaction space R. To put it in more detail, it is desirable to select an AC voltage having a frequency in the range 1 kHz to 500 MHz for the AC electric field.

In addition, in the selective disassociation process, an enzyme determined in advance can be added to the reaction space R in order to promote the process to select the mismatching double-standard nucleic acid 2 is to be disassociated (enzyme process). As an enzyme to be used in the enzyme process, an enzyme for recognizing the mismatching member of the mismatching double-standard nucleic acid 2 and cutting off the member from the mismatching double-standard nucleic acid 2 is appropriate. If the mismatching double-standard nucleic acid 2 is a double-strand DNA for example, Endonuclease V for recognizing a one-base mismatching member of the mismatching double-standard nucleic acid 2 and cutting off the member from the mismatching double-standard nucleic acid 2 can be used as an appropriate enzyme.

If an enzyme such as Endonuclease V is used for cutting off the single-base mismatching member from the mismatching double-standard nucleic acid 2, the mismatching double-standard nucleic acid 2 is divided into two double-strand portions, which are each shorter then the mismatching double-standard nucleic acid 2. As a result, the Tm decreases to a temperature lower than the Tm of the mismatching double-standard nucleic acid 2. Thus, in comparison with undivided mismatching double-standard nucleic acid 2, the two double-strand portions can each be subjected to a selective disassociation process at a temperature in a lower-temperature range. Thus, if the temperature increasing process is carried out to raise the temperature of the reaction space R to a temperature not exceeding an upper limit lower than the Tm of the fully-matching double-strand nucleic acid 1 along with execution of the process to apply an electric field to the reaction space R, the two double-strand portions obtained as a result of dividing the mismatching double-standard nucleic acid 2 can each be split into single-strand nucleic acids in the selective disassociation process.

### (Cleaning Process to Remove a Disassociated Single-Strand Nucleic Acid)

Refer back to Fig. 3. A subsequent cleaning process is carried out in order to remove the disassociated single-strand nucleic acid (denoted by notation 2b in Fig. 3), which exists in the reaction space R as a nucleic acid resulting from the temperature increasing process and/or the electric-field applying process or these processes as well as the enzyme process, from the reaction space R. For example, in the cleaning process to remove the disassociated single-strand nucleic acid from the reaction space R, a cleaning buffer solution with precisely set conditions is introduced into the reaction space R. The precisely set conditions include pH, temperature and concentration conditions, which do not have an effect on the complementary junction of the fully-matching double-strand nucleic acid 1. The target single-strand nucleic acid is removed from the reaction space R along with the buffer solution. It is to be noted that the middle diagram of Fig. 3 shows a model of a state of removing the target single-strand nucleic acid 2b, which has been disassociated from the mismatching double-standard nucleic acid 2, from the reaction space R.

### (Intercalater Supplying Process)

Next, an intercalater I is supplied to the reaction space R in which the fully-matching double-strand nucleic acid 1 and the probe nucleic acid 2a of the mismatching double-standard nucleic acid 2 remain. The intercalater I is not limited to any particular substance but can be any substance as long as the substance exhibits a property of generating a fluorescence beam when bound in a peculiar way to the double-strand member or absorbed by the member. Keep in mind that, of course, it is desirable to rather make use of an intercalater I, which is not bound in a non-peculiar way to a portion other than the double-strand member or absorbed by the portion in as many intercalater supplying processes as possible.

### (Fluorescence intensity Detection Process)

After a time period determined in advance has lapsed since the process to supply an intercalater I to the reaction space R, a fluorescence excitation light Q is generated by an external light source such as a laser light source for generating a laser light beam having a wavelength determined in advance and converged to be radiated to the reaction space R as a fluorescence excitation light Q for exciting the intercalater I to emit an excitation fluorescence F. A fluorescence intensity representing the intensity of the excitation fluorescence F is then measured by making use of light detection means D such as the commonly known photo detector in order to detect hybridization.

In this case, an excitation fluorescence F₁ generated by an intercalater I₁ bound to or absorbed by the fully-matching double-strand nucleic acid 1 is a fluorescence beam useful for detection of a normal hybridization in the reaction space R. In this process to measure the fluorescence intensity, the disassociated target single-strand nucleic acid 2b (2b₁ or 2b₂) existing in a free state has been removed from the reaction space R in the cleaning process carried out previously. Thus, no noise fluorescence beam is generated from an intercalater absorbed in a non-peculiar way by the disassociated target single-strand nucleic acid 2b existing in a free state. As a result, the S/N ratio can be improved.

It is quite within the bounds of possibility, however, that an intercalater I₂ is non-particularly absorbed by the probe nucleic acid 2a remaining (or fixed) in the reaction space R in a single-strand state even after execution of the cleaning process to remove the target single-strand nucleic acid 2b from the reaction space R, and generates a noise excitation fluorescence F₂ as shown in the top diagram of Fig. 3. Thus, at the stage of carrying out the process of measuring the fluorescence intensity, it is desirable to remove the probe nucleic acid 2a from the reaction space R in advance. A proper typical method for removing the probe nucleic acid 2a from the reaction space R is explained as follows.

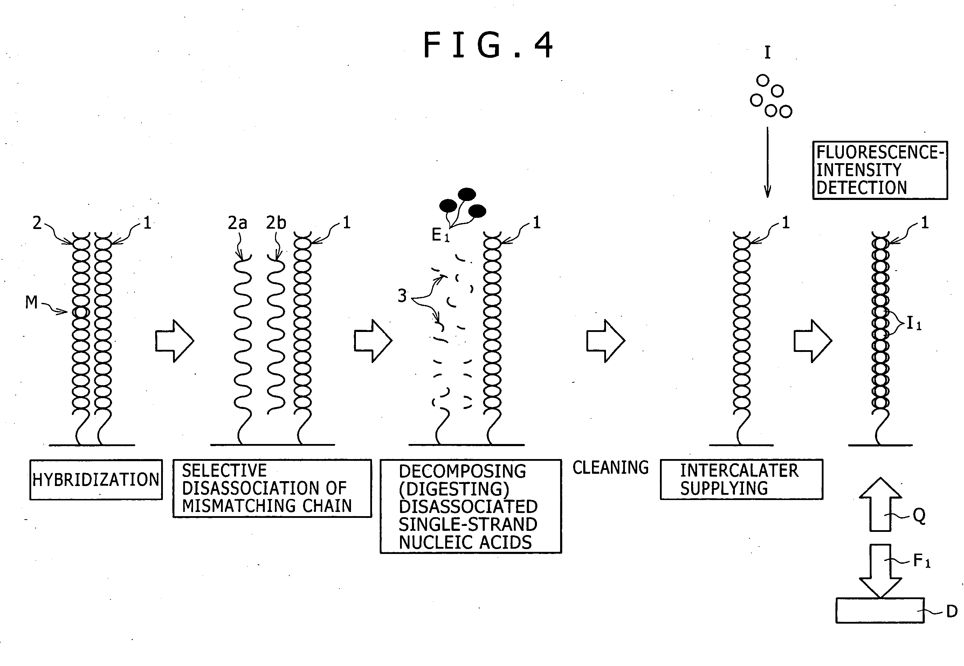

Fig. 4 is a flow diagram showing the process flow of a second typical hybridization detection method provided by the present invention.

Processes carried out in accordance with the a second typical hybridization detection method are divided into main processes, which are sequentially listed as follows:
a hybridization process of carrying out hybridization;
a selective disassociation process of disassociating an unnecessary target single-strand nucleic acid from a selected mismatching double-strand nucleic acid (abbreviated to a mismatching chain);
a process of decomposing (or digesting) disassociated single-strand nucleic acids and removing the decomposed single-strand nucleic acids from the reaction space (if necessary)
an intercalater supplying process; and
a fluorescence-intensity measuring process of detecting the hybridization.

The hybridization process and the selective disassociation process following the hybridization process are exactly the same as those of the first typical hybridization detection method explained above by referring to Fig. 3. Thus, the descriptions or the hybridization process and the selective disassociation process are not repeated in order to avoid duplications. The remaining processes of the listed above are explained sequentially as follows.

### (Process of Decomposing (or Digesting) Disassociated Single-Strand Nucleic Acids)

This process is carried out in order to decompose (or digest) single-strand nucleic acids existing in the reaction space R in single-base units at a stage prior to the fluorescence-intensity measuring process by making use of an enzyme E1 determined in advance. If not decomposed, the single-strand nucleic acids will non-peculiarly absorb an intercalater I, which will undesirably serve as a source generating a noise fluorescence beam. In addition, in some cases, such a single-strand nucleic acid forms a self-loop structure and, if an intercalater I is bound to this structure portion or absorbed by the portion, the intercalater I will undesirably generate a noise fluorescence beam. Also from this point of view, it is necessary to decompose the single-strand nucleic acids by making use of the enzyme E₁.

It is to be noted that, in this process of decomposing (or digesting) single-strand nucleic acids, the single-strand nucleic acids to be decomposed at least include:
(1): the probe nucleic acid 2a generated as a disassociated nucleic-acid chain by a temperature increasing process and an electric-field applying process, which are carried out to perform a selective disassociation process for the mismatching double-standard nucleic acid 2;
(2): the target single-strand nucleic acid 2b generated as a disassociated nucleic-acid chain by a temperature increasing process and an electric-field applying process, which are carried out to perform a selective disassociation process for the mismatching double-standard nucleic acid 2;
(3): a single-strand nucleic acid generated as a result of a temperature increasing process and an electric-field applying process, which are carried out after an enzyme process making use of an enzyme such as Endoniclease V is performed;
(4): a target nucleic-acid chain and/or a mismatching nucleic-acid chain, which have been excessively added to the reaction space R and hence exist in a free state (not shown in the figure); and
(5): a probe nucleic-acid chain not contributing to the hybridization.

The enzyme E₁ that can be used in the process of decomposing (or digesting) single-strand nucleic acids is an enzyme for decomposing the single-strand nucleic acids in a peculiar way. Examples of such an enzyme are the endonuclease, the exonuclease and the T4 DNA polymerase.

A result of the process of decomposing (or digesting) single-strand nucleic acids is a state in which a group of nucleic acid molecules 3 of single-base units and the fully-matching double-strand nucleic acid 1 exist as shown in the middle diagram of Fig. 4. It is to be noted that, after the process of decomposing (or digesting) single-strand nucleic acids is executed, a cleaning process to remove the group of nucleic acid molecules 3 from the reaction space R may be carried out just to make sure that the reaction space R is clean. In this way, the single-strand nucleic acids are removed from the reaction space R with a high degree of reliability as shown in Fig. 4.

### (Intercalater Supplying Process and Fluorescence Intensity Measuring Process)

With the single-strand nucleic acids removed from the reaction space R, an intercalater I is supplied to the reaction space R in which the fully-matching double-strand nucleic acid 1 exists. Then, after a time period determined in advance has lapsed, a fluorescence excitation light Q is generated by an external light source such as a laser light source for generating a laser light beam having a wavelength determined in advance and converged to be radiated to the reaction space R as a fluorescence excitation light Q for exciting the intercalater I to emit an excitation fluorescence F₁. A fluorescence intensity representing the intensity of the excitation fluorescence F is then measured by making use of light detection means D such as the commonly known photo detector in order to detect hybridization.

It is to be noted that, in accordance with the present invention, prior to the fluorescence-intensity measuring process or at the stage of measuring the fluorescence intensity, a protein process may be carried out in order to decompose a self-loop structure possibly created by a single-strand nucleic acid existing in the reaction space R so as to restore the normal single-strand state. Such a protein process is useful for preventing the intercalater I from being bound to the self-loop structure or absorbed by the self-loop structure, the intercalater I from generating a noise fluorescence beam and, thus, the S/N ratio from undesirably deteriorating. A protein suitable for this process is a protein demonstrating an effect of decomposing a self-loop structure created by a single-strand nucleic acid and restoring the normal single-strand state.

For example, a single-strand DNA binding protein abbreviated hereafter to an SSB can be used in the protein process. The SSB is a protein indispensable to a process of copying a DNA. By virtue of the effect demonstrated by the SSB as an effect of decomposing a self-loop structure, the intercalater I is released from the self-loop structure to the reaction space R so that a light beam (a noise fluorescence beam) that would otherwise be generated by the intercalater I can be eliminated.

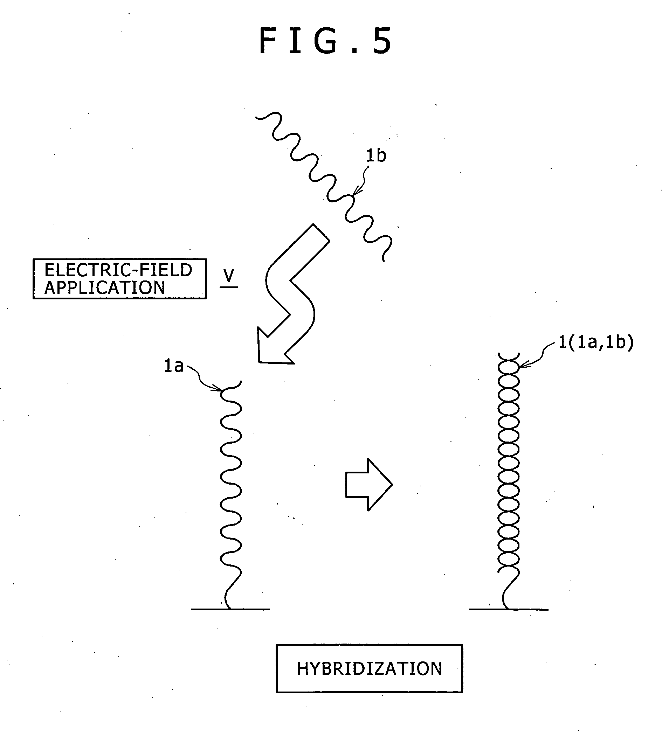

Fig. 5 is a diagram referred to in description of the concept an additional method that can be adopted in the stage of carrying out hybridization process according to the hybridization detection method provided by the present invention.

First of all, it is an aim of the additional method to increase the hybridization quantity in the reaction space R and improve the measurement sensitivity at the stage of measuring the fluorescence intensity by raising the hybridization efficiency.

In accordance with the additional method to be explained by referring to Fig. 5, at the stage of advancing hybridization, an electric field such as an alternating current (AC) electric field is applied to the reaction space R. By virtue of an electrodynamical effect obtained as a result of applying the electric field under a proper condition, a target nucleic-acid chain 1b is moved to the vicinity of a probe nucleic-acid chain 1a so as to promote hybridization. Examples of the electrodynamical effect are a dielectrophoresis and an electrophoresis.

For example, the dielectrophoresis in the reaction space R can be used. It is to be noted that the dielectrophoresis is a phenomenon showing a molecule driven in a direction toward a stronger electric field at a place with a non-uniform electric field. Even if an AC voltage is applied, inversion of the polarity of the applied voltage is followed by inversion of the polarity of the electric-field polarization. Thus, the same driving effect as the direct current (DC) voltage is obtained. In particular, in a high-frequency AC electric field, a force proportional to the gradient of the second power of a time-axis electric-field average is applied to each of the two poles, resulting in an electrophoresis. For example, it is known that, when a nucleic acid molecule experiences the effect of an electric field at a liquid phase, the nucleic acid molecule extends or moves. As its principle, it is conceivable that an ion cloud is created by phosphorous acid ions (with negative electric charge) forming a skeleton and hydrogen atoms (with positive electric charge) obtained as a result of ionization of hydrogen existing in the surroundings of the phosphorous acid ions. A polarization vector (or a two-pole vector) generated by the negative electric charge and the positive electric charge is, as a whole, oriented in one direction due to application of a high-frequency high-level voltage. As a result, the nucleic acid molecule is stretched. In addition, if a non-uniform electric field is applied, the nucleic acid molecule moves to a portion at which electric-force lines are concentrated. For more information, refer to "Quantitative analysis on electrostatic orientation of DNA in stationary AC electric field using fluorescence anisotropy" authored by Seichi Suzuki, Takeshi Yamanashi, Shin-ichi Tazawa, Osamu Kurosawa and Masao Washizu, IEEE Transaction on Industrial Applications, Vol. 34, No. 1, P75 to 83 (1998).

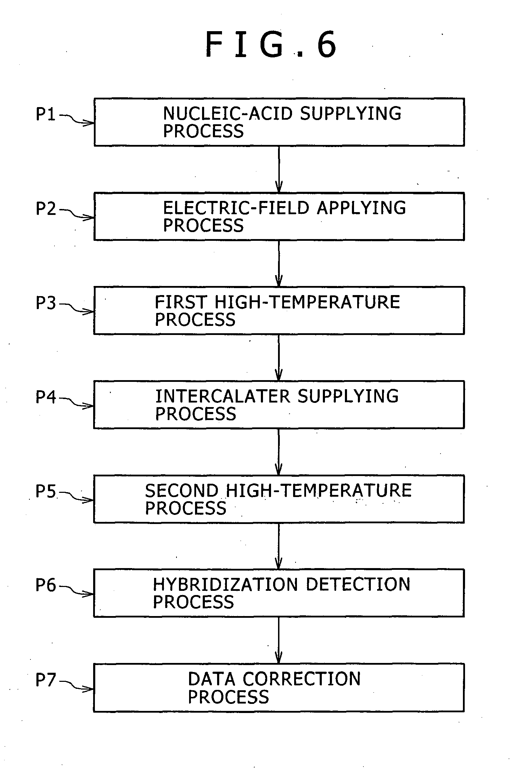

Fig. 6 is a flow diagram showing the process flow of a third typical implementation of the hybridization detection method provided by the present invention.

The hybridization detection method represented by the process flow shown in Fig. 6 includes:
a nucleic-acid supplying process denoted by notation P1 as a process of supplying a sample nucleic acid to the reaction space R in which a probe nucleic-acid chain is held or fixed;
an electric-field applying process denoted by notation P2 as a process of applying an electric field to the reaction space;
a first high-temperature process denoted by notation P3 as a process of raising the temperature of the reaction space;
an intercalater supplying process denoted by notation P4 as a process of supplying an intercalater to the reaction space;
a second high-temperature process denoted by notation P5 as a process of raising the temperature of the reaction space;
a hybridization detection process denoted by notation P6 as a process of detecting hybridization; and
a data correction process denoted by notation P7 as a process of correcting hybridization detection data on the basis of hybridization detection data obtained previously.

It is to be noted that implementations of the hybridization detection method provided by the present invention are by no means limited to the third typical implementation described above. That is to say, some of the processes listed above can be combined properly in accordance with the objective of the hybridization detection method, and such a combination is also included in the present invention.

### Nucleic-Acid Supplying Process (P1)

As described earlier, the nucleic-acid supplying process denoted by notation P1 is a process of dropping or supplying a sample nucleic acid to the reaction space in which a probe nucleic-acid chain is held or fixed. For example, an automatic process is carried out to continuously supply a sample having a quantity determined in advance from a nozzle or the like to a well created on the substrate surface of a DNA chip to serve as a reaction space.

### Electric-Field Applying Process (P2)

The electric-field applying process denoted by notation P2 is a process of applying an electric field to the reaction space R in order to move a target nucleic-acid chain to the vicinity of a probe nucleic-acid chain as shown in Fig. 5. The electric-field applying process can be any arbitrary process. By carrying out the electric-field applying process P2, hybridization can be promoted. Thus, the precision of the detection of the hybridization can be improved.

### First High-Temperature Process (P3)

The first high-temperature process denoted by notation P3 is a process of raising the temperature of the reaction space to a first temperature lower than the melting temperature Tm of the target nucleic-acid chain in order to advance the hybridization till a state of a reaction equilibrium at the first temperature is reached. Then, in this first high-temperature process, the temperature of the reaction space is further raised to a second temperature higher than the melting temperature Tm of a mismatching double-strand nucleic acid obtained as a result of a mis-hybridization but lower than the melting temperature Tm of a fully matching double-strand nucleic acid obtained as a result of a normal hybridization in order to selectively carry out a disassociation process on the mismatching double-strand nucleic acid only. Thus, the mis-hybridization quantity relative to the quantity of the hybridization can be reduced so as to increase the precision of the detection of the hybridization. It is to be noted that a part or all of the first high-temperature process P3 can be carried out concurrently with electric-field applying process P2.

### Intercalater Supplying Process (P4)

The intercalater supplying process denoted by notation P4 is a process of supplying an intercalater I to the reaction space. If the intercalater I is an intercalater to be bound in a peculiar way to a double-strand nucleic acid. The type of the intercalater I is not limited in particular to a specific one. By making use of an intercalater I, the hybridization can be detected by measuring the intensity of a fluorescence beam generated by the intercalater I. That is to say, the hybridization can be measured quantitatively.

### Second High-Temperature Process (P5)

The second high-temperature process denoted by notation P5 is a process of raising the temperature of the reaction space R to a temperature higher than a temperature determined in advance in order to disassociate an intercalater I from a single-strand nucleic acid put in a state of already absorbing the intercalater I and decompose the self-loop structure of a single-strand nucleic acid already bound to an intercalater I. By carrying out the second high-temperature process P5, the magnitude of a fluorescence noise (or a background noise) can be reduced in the process of detecting the hybridization by making use of an intercalater I. Thus, the S/N ratio seen at the hybridization detection time can be raised and the precision of the detection of the hybridization can be improved.

### Hybridization Detection Process (P6)

As described earlier, the hybridization detection process denoted by notation P6 is a process of detecting hybridization between a nucleic-acid probe and a target nucleic acid by measuring a fluorescence intensity.

### Data Correction Process (P7)

The data correction process denoted by notation P7 is a process of correcting hybridization detection data on the basis of hybridization detection data obtained previously. For example, fluorescence intensities before and after execution of a temperature increasing process are acquired in advance for each measurement. Then, on the basis of the acquired fluorescence intensities, a correction constant representing the degree to which the S/N ratio has been raised is computed. Finally, by making use of the correction coefficient, the hybridization detection data is corrected. Thus, the precision of the detection of the hybridization can be further improved.

### First Embodiment

A first embodiment was implemented in order to examine whether or not the quantity of the mis-hybridization could be reduced and, hence, the S/N ratio of the hybridization could be raised by executing the high-temperature processing procedure after the execution of the hybridization. An outline of this experimental procedure according to the first embodiment is explained as follows.

First of all, a substrate to be used in an experiment was made. A silicon substrate cut off to a 1-cm angle was cleaned and subjected to ozone processing and over-oxidized hydrogen hydro processing in order to subject the surface of the substrate to a cleaning and hydroxiding process. Then, the silicon substrate was subjected to a heating and drying process carried out at a temperature of 20 for 30 minutes. Subsequently, an amino propyl silane was added to a toluene with the volume of the former set at 2% of the latter in order to prepare a solution. Then, the cleaned substrate was submersed into the solution set at a temperature of 50 for 30 minutes in order to let a hydroxyl group on the silicon surface have a reaction with the amino propyl silane. Subsequently, the silicon substrate was cleaned by making use of toluene and water. Then, a succinic acid anhydride having a weight of 100 mg was dissolved in a dimethylformamide having a volume of 10 ml in order to prepare a solution and the substrate was submersed into the solution in order to execute an amide-conversion reaction at a temperature of 50 for 2 hours. In this way, the amino propyl silane had a reaction with the amber acid anhydride, and the substrate was cleaned.

Then, a DNA oligomer was fixed on the surface of the substrate. After the DNA oligomer (with a base sequence number of 1) having an amino end was dissolved in pure water to result in a solution having a concentration of 10 µM, the solution was dropped on a 1 mm spot on the surface of the substrate in order to let an amino group of the DNA oligomer have a reaction with a succinic acid on the surface of the substrate at an increased humidity and a temperature of 80 for 1 hour so as to fix the DNA oligomer.

Then, hybridization between the probe nucleic-acid chain fixed on the surface of the substrate and the target nucleic-acid chain included in the sample nucleic acid was measured. The sample nucleic acid was a nucleic acid (a fully matching nucleic acid with a base sequence number of 2) having a base sequence complementary to the entire base sequence of the probe nucleic-acid chain, and a nucleic acid (a mismatching nucleic acid with a base sequence number of 3) having a base sequence complementary to only a part of the base sequence of the probe nucleic-acid chain. The sample nucleic acid was dissolved in a Hepes buffer in order to prepare a solution with a concentration of 1 nM and, then, the solution was heaped up as a liquid having a volume of 10 µL on the surface of the substrate. Subsequently, the hybridization was advanced at an increased humidity and a temperature of 65 for 4 hours. After the hybridization reaction was performed, a higher-temperature process was carried out at a temperature of 80 for 5 minutes and SYBR Green I was added as an intercalater and an exiting light beam of 450 nm was radiated. Then, the excited fluorescence intensity of the substrate was measured. In this case, by SYBR Green I, an intercalater made by Molecular Probes Corporation is meant and, in addition, an intercalater mentioned in the following description is also an intercalater made by Molecular Probes Corporation. It is to be noted that, in a radiated region, after the hybridization reaction was performed, the higher-temperature process set at a temperature of 80 to last 5 minutes was not carried out. In this case, the fluorescence intensity of the substrate was measured by making use of a microscope.

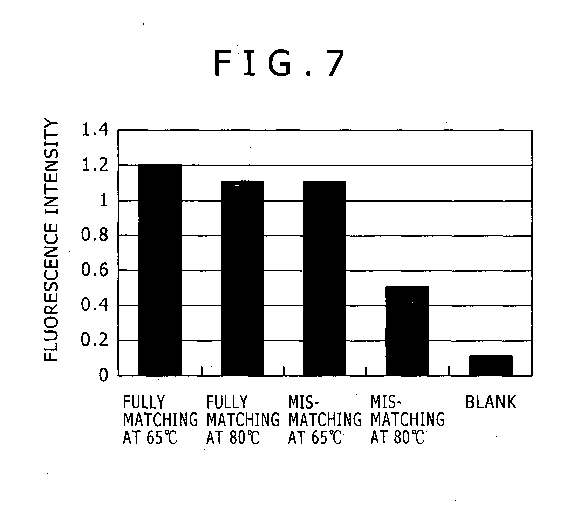

Results of the experiment are shown in Fig. 7. In the figure, the vertical axis represents the relative value of a fluorescence intensity. The leftmost bar graph represents a fluorescence intensity measured by making use of a fully matching nucleic acid without carrying out the higher-temperature process. The second bar graph from the left represents a fluorescence intensity measured by making use of the fully matching nucleic acid after carrying out the higher-temperature process. The third bar graph from the left represents a fluorescence intensity measured by making use of a mismatching nucleic acid without carrying out the higher-temperature process. The fourth bar graph from the left represents a fluorescence intensity measured by making use of the mismatching nucleic acid after carrying out the higher-temperature process. The rightmost bar graph represents a fluorescence intensity measured in a blank state without adding a sample nucleic acid and without carrying out the higher-temperature process.

As shown in Fig. 7, in the case of a fully matching nucleic acid, the ratio of a fluorescence intensity measured without carrying out the higher-temperature process to a fluorescence intensity measured after carrying out the higher-temperature process is 1.2 : 1.1. That is to say, even if the higher-temperature process is carried out, the fluorescence intensity does not decrease that much. In the case of a mismatching nucleic acid, on the other hand, the ratio of a fluorescence intensity measured without carrying out the higher-temperature process to a fluorescence intensity measured after carrying out the higher-temperature process is 1.1 : 0.5. That is to say, with the higher-temperature process carried out, the fluorescence intensity decreases substantially.

The experiment results described above reveal that, by carrying out the higher-temperature process after execution of hybridization, the detected quantity of a mis-hybridization can be reduced and the ratio of a fluorescence intensity measured by making use of a fully matching nucleic acid to a fluorescence intensity measured by making use of a mismatching nucleic acid can be increased considerably. That is to say, the experiment results described above reveal that, by reducing the detected quantity of the mis-hybridization, the S/N ratio seen at hybridization detection time can be increased so that the precision of the detection of the hybridization can be improved.

### Second Embodiment

In the case of a second embodiment, a higher-temperature processing procedure was carried out after hybridization was performed in the same way as the first embodiment in order to examine whether or not the quantity of the mis-hybridization could be reduced and, hence, the S/N ratio of hybridization could be increased. It is to be noted that, in an experiment according to the second embodiment, hybridization chromatophore Cy3 was bound to a sample nucleic acid in order to measure the fluorescence intensity at hybridization detection time.

An outline of this experimental procedure according to the second embodiment is explained as follows.

First of all, an avidin was fixed on the surface of a substrate. A jean silicon (a registered trademark of Toyo Kohan Corporation was submersed in an avidin solution (referred to as a HEPES Buffer having a weight of 100 mg/l) having a volume of 300 µL at a room temperature for 30 minutes in order to fix the avidin. Then, the HEPES Tween Buffer was used for cleaning lasting 5 minutes. The HEPES Buffer was replaced three times.

Then, a probe fixing process was carried out. A DNA oligomer was subjected to a biotin process in order to prepare a probe fixing solution in advance. Then, a substrate was submersed in the probe fixing solution having a volume of 300 µL, that is, a substrate was submersed in a HEPES Buffer with a concentration of 500 nM, at a room temperature for 60 minutes in order to fix the DNA oligomer by an avidin-biotin junction. Subsequently, the HEPES Tween Buffer was used for cleaning lasting 5 minutes. The HEPES Buffer was replaced three times.

Then, hybridization between the probe nucleic-acid chain fixed on the surface of the substrate and a target nucleic acid included in a sample nucleic acid was measured. As the sample nucleic acid, a 30-mer fully matching nucleic acid and a 30-mer mismatching nucleic acid were used. The 30-mer fully matching nucleic acid was a nucleic acid having a base sequence complementary to the entire base sequence of the probe nucleic-acid chain. On the other hand, the 30-mer mismatching nucleic acid was a nucleic acid having a base sequence complementary to the base sequence of all the probe nucleic-acid chain, except 3 bases of the sequence. The sample nucleic acid was dissolved in a HEPES Buffer in order to prepare a solution having a concentration of 50 nM. Then, after a chromatophore Cy3 was bound to the sample nucleic acid, the solution of the sample nucleic acid was poured on a multi-dish well in 300 µL units. Subsequently, after a substrate was submersed in the solution of the sample nucleic acid, the solution was subjected to an incubation process in hybridization oven at a temperature of 55 for 3 hours in order to advance hybridization. After the hybridization reaction was performed, a HEPES Tween Buffer was used for cleaning lasting 5 minute's. The HEPES Buffer was replaced three times. Then, after the hybridization reaction was performed, a high-temperature process was carried out at a temperature of 80 for 5 minutes. Subsequently, the substrate was set under a fluorescence intensity microscope for measuring the fluorescence intensity. It is to be noted that, in a radiated region, the higher-temperature process set at a temperature of 80 to last 5 minutes was not carried out after the hybridization reaction was performed. In this case, the fluorescence intensity of the substrate was also measured by making use of the microscope.

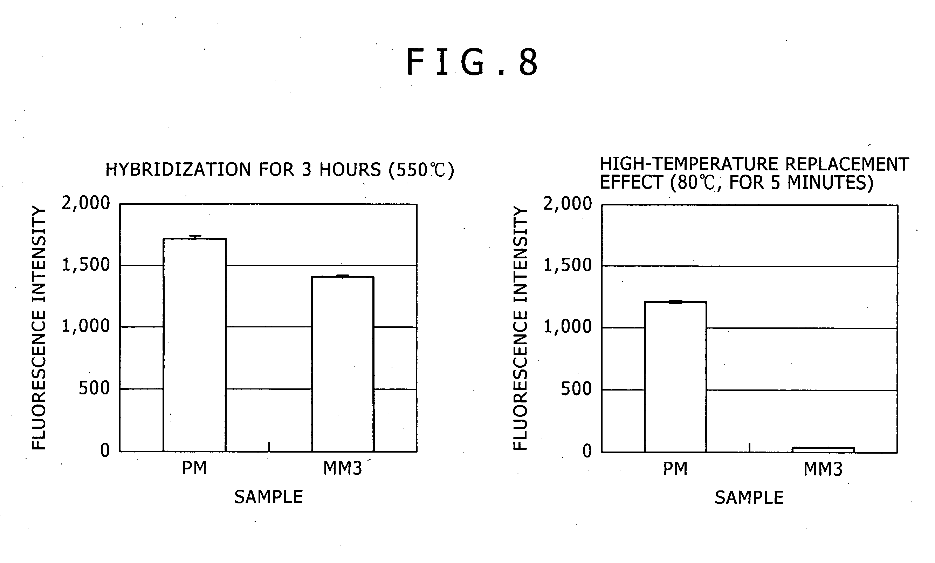

Results of the experiment are shown in Fig. 8. The left-side diagram shows results obtained by carrying out no high-temperature process whereas the right-side diagram shows results obtained by carrying out a high-temperature process. A fluorescence intensity ratio (MM3/PM) is defined as the ratio of the fluorescence intensity obtained for a mismatching nucleic acid to the ratio of the fluorescence intensity obtained for a fully matching nucleic acid. In the case of the results shown in the left-side diagram, the fluorescence intensity ratio is 0.82 whereas, in the case of the results shown in the right-side diagram, the fluorescence intensity ratio is 0.03. That is to say, by carrying out a high-temperature process after hybridization was executed, the S/N ratio of the fluorescence intensity could be improved by at least 25 times.

Much like the first embodiment, the experiment results described above reveal that, by carrying out the higher-temperature process after hybridization is executed, the detected quantity of a mis-hybridization can be reduced and the ratio of a fluorescence intensity measured by making use of a fully matching nucleic acid to a fluorescence intensity measured by making use of a mismatching nucleic acid can be increased considerably. That is to say, the experiment results described above reveal that, by reducing the detected quantity of the mis-hybridization, the S/N ratio seen at hybridization detection time can be increased so that the precision of the detection of the hybridization can be improved.

### Third Embodiment

In the case of a third embodiment, after an intercalater was bound to a double-strand nucleic acid, the procedure of the high-temperature process was carried out in order to examine whether or not the quantity of the mis-hybridization could be reduced and, hence, the S/N ratio of the hybridization could be raised.

The procedure of an experiment according to the third embodiment is explained as follows. A DNA oligomer (or a probe nucleic-acid chain) having a concentration of 150 nM, DNA oligomers (a target nucleic-acid chain and a sample nucleic acid not used as a target) having a concentration of 150 nM and an intercalater (or SYBR (a registered trademark) Green I) were submersed in hybridization buffer in order to prepare a solution. The target nucleic-acid chain is complementary to the probe nucleic-acid chain but the sample nucleic acid not used as a target is not complementary to the probe nucleic-acid chain. Then, after the solution was subjected to a high-temperature process carried out at a temperature of 95 for 5 minutes in order to convert each of the nucleic acids into a single-strand nucleic acid, the solution was abruptly cooled to a temperature of 4. Subsequently, after an incubation process was carried out at a temperature of 25 for 1 minute, the intensity of a fluorescence beam generated by the intercalater was detected. Then, the temperature was increased gradually by 1 degree Celsius at a time and, at each temperature, an incubation process was carried out for 1 minute. By the same token, after an incubation process was carried out at each temperature for 1 minute, the intensity of a fluorescence beam generated by the intercalater was detected. It is to be noted that the combination of hybridization buffers used in the experiment was 100 mM HEPES (a pH of 7.7) and 20 mM MgCl₂. In addition, in this experiment, a DNA having a base sequence with a base sequence number of 5 was used as the probe nucleic-acid chain, a DNA having a base sequence with a base sequence number of 4 was used as the target nucleic acid and a DNA having a base sequence with a base sequence number of 6 was used as the sample nucleic acid, which was not a target.

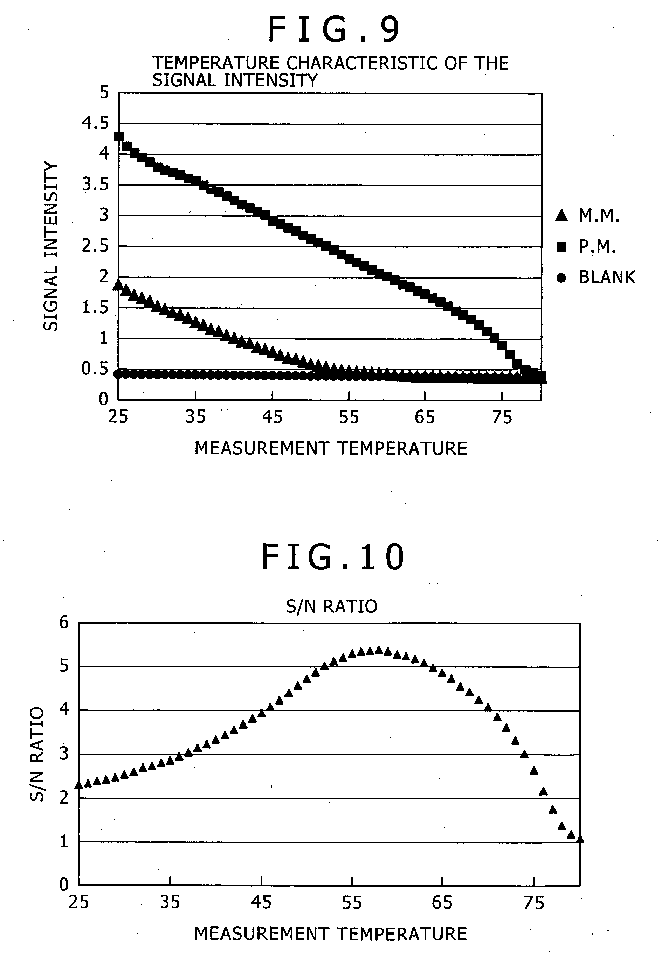

Results of the experiment are shown in Figs. 9 and 10. To be more specific, Fig. 9 is a diagram showing the temperature characteristic of the fluorescence intensity whereas Fig. 10 is a diagram showing the temperature characteristic of the S/N ratio computed on the basis of the fluorescence intensity shown in Fig. 9. In Fig. 9, the horizontal axis represents the temperature whereas the vertical axis represents the fluorescence intensity (or the signal intensity). In the same figure, an M.M. curve is a curve plotted for fluorescence intensity results obtained by making use of a sample nucleic acid not utilized as a target. On the other hand, a P.M. curve is a curve plotted for fluorescence intensity results obtained by making use of a target nucleic acid. A blank curve is a curve plotted for fluorescence intensity results obtained by adding no sample nucleic acid (or for results representing a controlled fluorescence intensity). In Fig. 10, on the other hand, the horizontal axis represents the temperature whereas the vertical axis represents the S/N ratio.

As shown in Fig. 10, if a probe nucleic-acid chain and a nucleic acid not complementary to the probe nucleic-acid chain (or a nucleic acid not used as a target) are added to hybridization buffer, the fluorescence intensity (represented by the M. M. curve) attenuates to a value of the same order as the blank curve for temperatures higher than about 55. Since a sample nucleic acid not used as a target is not involved in hybridization with a probe nucleic-acid chain, such a sample nucleic acid can be assumed to be the source of a fluorescence noise (or a background noise). That is to say, since such a sample nucleic acid forms a self loop and is bound to an intercalater or absorbed in a non-peculiar way by an intercalater, the fluorescence intensity of the sample nucleic acid can be assumed to have been detected. Thus, the experiment results strongly suggest that, in order to detect hybridization by making use of an intercalater, after the intercalater is supplied, a process to increase the measurement temperature to at least 55 be carried out in order to reduce the intensity of the fluorescence noise.

In addition, also as shown in Fig. 10, if hybridization is detected by making use of an intercalater, the S/N ratio reaches a maximum value at a temperature close to 58. As the measurement temperature increases, the hybridization between the probe nucleic-acid chain and the target nucleic acid is gradually decomposed. Thus, for excessively high temperatures, the S/N ratio adversely decreases. That is to say, the experiment results suggest that, in order to detect hybridization by making use of an intercalater, a process to increase the measurement temperature around 58 be carried out in order to increase the S/N ratio and, hence, improve the precision of the detection of the hybridization.

### Fourth Embodiment

A fourth embodiment is an embodiment implementing a simulation of a temperature increasing process carried out in order to examine whether or not a nucleic acid already subjected to a mis-hybridization can be disassociated from a probe nucleic acid.

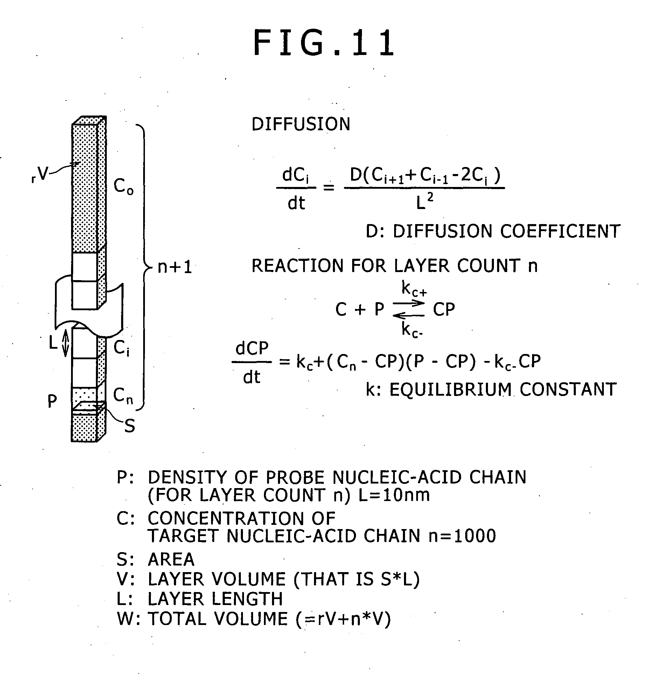

Fig. 11 is a diagram showing a model of the simulation. In this model of the simulation, n unit computation cells each having a layer height L are stacked on a substrate S to form a diffusion layer Cₙ. A bulk layer Cₒ exists above the diffusion layer Cₙ.

A probe nucleic-acid chain P is assumed to exist on the surface of the substrate S and hybridization reaction is assumed to progress in the diffusion layer Cₙ in contact with the substrate S. It is also assumed that a movement of a target nucleic acid in the diffusion layer Cₙ is approximated by an equation on the upper side of the figure whereas the reaction speed of hybridization reaction CP taking place in the diffusion layer Cₙ is expressed by an equation on the lower side of the figure.

In actual calculations, the height (or the thickness) L of the diffusion layer Cₙ is 10 nm, a layer count n representing the number of cell layers is 1,000, the area (A) of a reaction space of the hybridization reaction CP on the surface S of the substrate is 10 nm² and the concentration P of the probe nucleic-acid chain is 1 × 10¹¹ / mm². In addition, the total volume W of the reaction space is 5 µL, the concentration C of the target nucleic acid is 1 nM for both the fully matching and mismatching nucleic acids, the diffusion constant D is 1 × 10⁹ and the temperatures of the reaction space are 65 and 80.

Since the reaction speed of the hybridization reaction CP making use of a fully matching nucleic-acid chain is all but equal to the reaction speed of the hybridization reaction CP making use of a mismatching nucleic-acid chain, the reaction speed was computed from an activation energy Ea⁺ (= 112 kcal / mol) and a frequency factor lnA (= 163), which are described in a reference referred to as "Biochem. 32 (1993) 3095". The speed of a reverse reaction was computed from an equilibrium constant (or a kinetic constant) found in accordance with a nearest neighbor method and the reaction speed of the hybridization reaction CP. In the case of a fully matching nucleic acid, an entropy ΔS was found in accordance with a nearest neighbor method to be -632.2 cal/mol whereas an enthalpy ΔH was found in accordance with a nearest neighbor method to be -231200 cal/mol. In the case of a mismatching nucleic acid, on the other hand an entropy ΔS was found in accordance with a nearest neighbor method to be -534 cal/mol whereas an enthalpy ΔH was found in accordance with a nearest neighbor method to be -198,000 cal/mol.

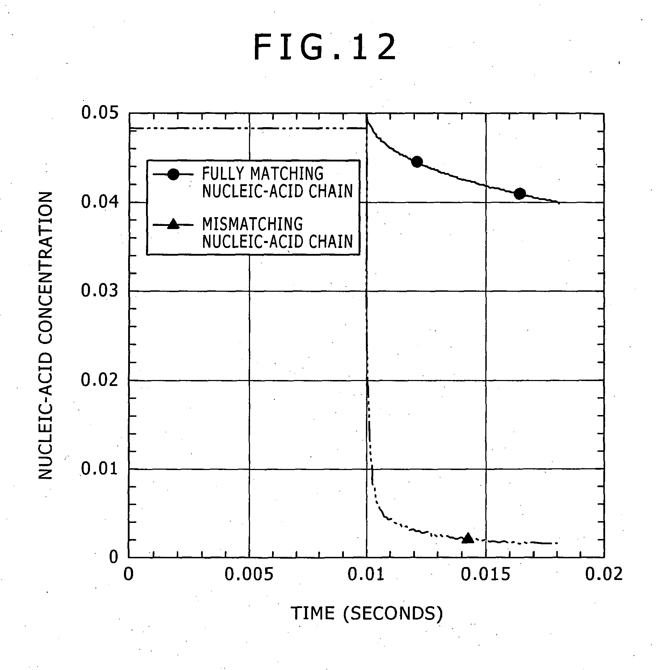

Fig. 12 is a diagram showing results of a simulation for a process of executing hybridizations for a fully matching nucleic acid and a mismatching nucleic acid at a temperature of 65 and then raising the temperature to 80. The vertical axis represents the concentration (expressed in terms of mol / m³) of a nucleic acid already subjected to hybridization on a substrate whereas the horizontal axis represents the hybridization lapsing time (expressed in terms of seconds). In Fig. 12, a curve plotted on circular marks represents results of a simulation for a process of executing hybridization for a fully matching nucleic acid whereas a curve plotted on triangular marks represents results of a simulation for a process of executing hybridization for a mismatching nucleic acid.

As shown in Fig. 12, for a reaction-space temperature set at 65 or for the period 0 to 0.01 seconds, the hybridization quantity for the fully matching nucleic acid is all but equal to the hybridization quantity for the mismatching nucleic acid. As the temperature of the reaction space is raised to 80 in a range after a point of 0.01 seconds, however, the concentration of the fully matching nucleic acid completing the hybridization does not decrease that much but the concentration of the mismatching nucleic acid completing the hybridization decreases substantially as shown by the curve on the lower side of Fig. 12.

Thus, the simulation results indicate that, by carrying out a temperature increasing process, the concentration of the mismatching nucleic acid completing the hybridization can be reduced substantially in an extremely short period of time. In addition, by carrying out a high-temperature process, only the mismatching nucleic acid can be disassociated.

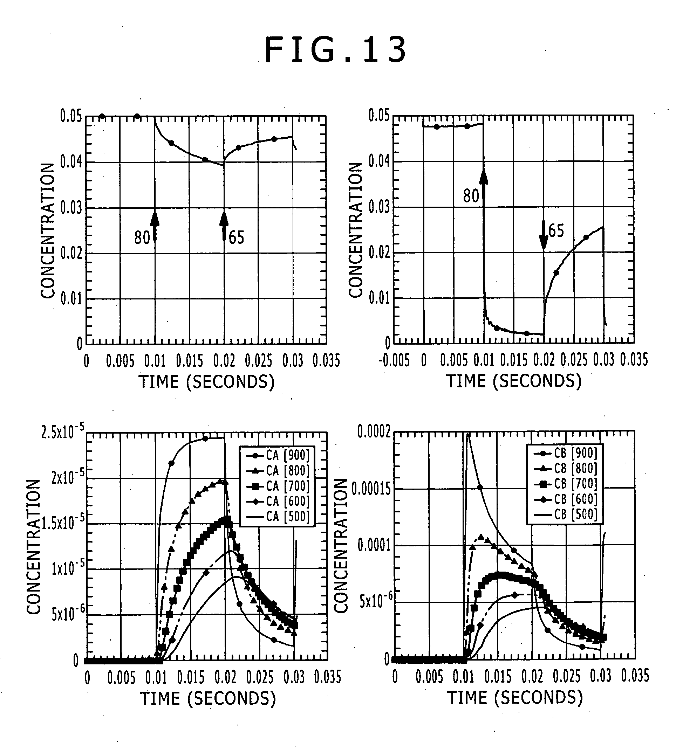

Fig. 13 is diagrams showing simulation results representing relations between hybridization quantities and temperatures of a reaction space as well as relations between concentrations of a target nucleic acid existing in a diffusion layer and temperatures of a reaction space for hybridizations making use of fully matching and mismatching nucleic acids.

A simulation result shown in the upper-left diagram of Fig. 13 represents a relation between variations in hybridization quantity and temperatures of a reaction space for hybridization carried out by making use of a fully matching nucleic acid. A simulation result shown in the upper-right diagram of Fig. 13 represents a relation between variations in hybridization quantity and temperatures of a reaction space for hybridization carried out by making use of a mismatching nucleic acid. In the case of both the diagrams, the vertical axis represents the concentration (expressed in terms of mol / m³) of a nucleic acid already subjected to hybridization on a substrate whereas the horizontal axis is the hybridization lapsing time (expressed in terms of seconds). Arrows shown in these diagrams each indicate a point of time at which the temperature of the reaction space is changed from 80 to 65 or vice versa.

A simulation result shown in the lower-left diagram of Fig. 13 represents a relation between concentrations of a target nucleic acid existing in a diffusion layer and temperatures of a reaction space for hybridization carried out by making use of a fully matching nucleic acid with the position of the target nucleic acid taken as a parameter. A simulation result shown in the lower-right diagram of Fig. 13 represents a relation between concentrations of a target nucleic acid existing in a diffusion layer and temperatures of a reaction space for hybridization carried out by making use of a mismatching nucleic acid with the position of the target nucleic acid taken as a parameter. In the case of both the diagrams, the vertical axis represents the concentration (expressed in terms of mol / m³) of a nucleic acid already subjected to hybridization on a substrate whereas the horizontal axis is the hybridization lapsing time (expressed in terms of seconds). Arrows shown in these diagrams each indicate a point of time at which the temperature of the reaction space is changed from 80 to 65 or vice versa in the same way as the upper simulation results shown in the upper diagrams. The position of the target nucleic acid taken as a parameter in both the simulation results represented by the CA and CB curves plotted for fully matching and mismatching nucleic acids respectively are described as follows. The CA [900] and CB [900] curves each represent concentrations of the target nucleic acid at a position separated away from the surface of the substrate by a distance of 1 µm. By the same token, the CA [800] and CB [800] curves each represent concentrations of the target nucleic acid at a position separated away from the surface of the substrate by a distance of 2 µm. In the same way, the CA [700] and CB [700] curves each represent concentrations of the target nucleic acid at a position separated away from the surface of the substrate by a distance of 3 µm. Likewise, the CA [600] and CB [600] curves each represent concentrations of the target nucleic acid at a position separated away from the surface of the substrate by a distance of 4 µm. Similarly, the CA [500] and CB [500] curves each represent concentrations of the target nucleic acid at a position separated away from the surface of the substrate by a distance of 5 µm.

As shown in the two upper diagrams of Fig. 13, in both the cases of the fully matching and mismatching nucleic acids used in the hybridization, when the temperature of the reaction space is increased to 80, the hybridization quantity decreases. As shown in the two lower diagrams of Fig. 13, in both the cases of the fully matching and mismatching nucleic acids used in the hybridization, when the temperature of the reaction space is increased to 80, the closer the position of the target nucleic-acid chain existing in the diffusion layer to the surface of the substrate, the more abrupt the way in which the concentration of the target nucleic acid increases.

As shown in the two upper diagrams of Fig. 13, when the temperature of the reaction space is increased, the hybridization quantity of the hybridization making use of a mismatching nucleic-acid chain decreases much more abruptly than the hybridization quantity of the hybridization making use of a fully matching nucleic-acid chain does. As shown in the two lower diagrams of Fig. 13, when the temperature of the reaction space is increased, the concentration of the target nucleic-acid chain existing in the diffusion layer in hybridization making use of a mismatching nucleic-acid chain increases much more abruptly than the concentration of the target nucleic-acid chain existing in the diffusion layer in hybridization making use of a fully matching nucleic-acid chain does. In addition, in hybridization making use of a mismatching nucleic-acid chain, the original hybridization concentration is not restored sufficiently in a short period of time even if the temperature of the reaction space is decreased back to 65.

The simulation results described above indicate that, by carrying out the temperature increasing process, the S/N ratio can be raised and, once the temperature increasing process is carried out, the state of the increased S/N ratio can be sustained even if the temperature of the reaction space is lowered again. In this case, the S/N ratio is defined as a ratio of the concentration of hybridization between a probe nucleic acid and a fully matching nucleic acid to the concentration of hybridization between the probe nucleic acid and a mismatching nucleic acid.

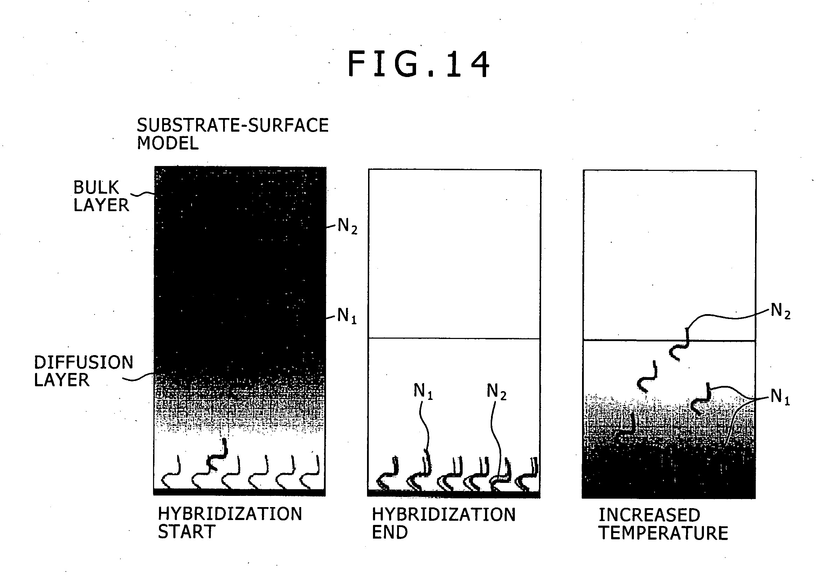

Fig. 14 is a diagram showing models of a behavior obtained from a simulation as the behavior of hybridization reaction.

At the start of hybridization, the fully matching nucleic acid denoted by notation N₁ in Fig. 14 advances the hybridization at about the same speed as a speed at which the mismatching nucleic acid denoted by notation N₂ in the same figure does. In addition, as the hybridization is advanced, a concentration gradient is created on the surface of the substrate as shown by the model on the left side of Fig. 14.

Then, when a reaction equilibrium state for the temperature of the reaction space is reached at the end of the hybridization, both the fully matching and mismatching nucleic acids carry out hybridizations with the probe nucleic-acid chain as shown by the model in the middle of Fig. 14.

Subsequently, by carrying out a temperature increasing process, a denature reaction is advanced. At that time, the disassociation speed of the hybridization between the probe nucleic acid and the mismatching nucleic acid N₂ his higher than the disassociation speed of the hybridization between the probe nucleic-acid chain and the fully matching nucleic acid N₁. Thus, the disassociation of the mismatching nucleic acid N₂ from the surface of the substrate and the diffusion of the disassociated mismatching nucleic acid N₂ take precedence of those of the fully matching nucleic acid N₁ as shown by the model on the right side of Fig. 14.

Accordingly, by carrying out a temperature increasing process after hybridization is advanced till a reaction equilibrium state for a predetermined temperature of the reaction space is reached, the quantity of a mis-hybridization can be reduced so that the S/N ratio can be increased. In this case, the mis-hybridization is hybridization between the probe nucleic-acid chain and the mismatching nucleic acid N₂.

### Fifth Embodiment

A fifth embodiment is an embodiment implementing execution of an experiment for verifying differences in effects on disassociation of a single-strand nucleic acid as seen from a mismatching-base-count point of view. The disassociation of a single-strand nucleic acid is disassociation caused by a temperature increasing operation performed to increase the temperature of the reaction space used for carrying out hybridization to generate a perfectly matching double-strand nucleic acid and mistakenly carrying out a mis-hybridization to inadvertently generate a mismatching double-strand nucleic acid. The perfectly matching double-strand nucleic acid is also referred to as a perfectly complementary double-strand nucleic acid. In this case, the temperature increasing operation is an operation to replace the present solution with a solution of a higher temperature. In the experiment, DNAs having mismatching base counts in the range 1 to 3 as described below were used in the experiment.

First of all, a target-DNA solution was introduced to a reaction space above a substrate on which a probe DNA had been fixed as described earlier. The target-DNA solution was a solvent including 100 mM HEPES and 200 mM NaCl whereas the probe DNA was a 30-mer DNA. Then, hybridization reaction was carried out at any arbitrary temperature, which was close to the melting temperature Tm of a DNA having a base sequence complementary to that of the probe DNA or lower than the melting temperature Tm.

Target-DNA solutions used in the experiment were solutions (1) to (4) described as follows:
(1): A solution obtained by mixing a DNA having a base sequence complementary to that of the probe DNA with a modified DNA at a mixing ratio of 1:1. The modified DNA is a DNA obtained as a result of modifying the DNA, which has a base sequence complementary to that of the probe DNA, by making use of the fluorescence chromatophore Cy3. The DNA having a base sequence complementary to that of the probe DNA is a DNA having a base sequence number of 4.
(2): A solution obtained by mixing a DNA having a base sequence complementary to that of the probe DNA with a modified DNA at'a mixing ratio of 1:1. The modified DNA in this case is a DNA obtained as a result of modifying the DNA, which has only one mismatching base included in its base sequence as a base complementary to that of the probe DNA, by making use of the fluorescence chromatophore Cy3. The DNA having only one mismatching base included in its base sequence as a base not complementary to that of the probe DNA is a DNA having a base sequence number of 5.
(3): A solution obtained by mixing a DNA having a base sequence complementary to that of the probe DNA with a modified DNA at a mixing ratio of 1:1. The modified DNA in this case is a DNA obtained as a result of modifying the DNA, which has only two mismatching bases included in its base sequence as bases not complementary to those of the probe DNA, by making use of the fluorescence chromatophore Cy3. The DNA having only two mismatching bases included in its base sequence as bases complementary to those of the probe DNA is a DNA having a base sequence number of 6.
(4): A solution obtained by mixing a DNA having a base sequence complementary to that of the probe DNA with a modified DNA at a mixing ratio of 1:1. The modified DNA in this case is a DNA obtained as a result of modifying the DNA, which has only three mismatching bases included in its base sequence as bases not complementary to those of the probe DNA, by making use of the fluorescence chromatophore Cy3. The DNA having only three mismatching bases included in its base sequence as bases complementary to those of the probe DNA is a DNA having a base sequence number of 7.

The base sequences of the fully matching target DNA and the three mismatching target DNAs described above are summarized in Table 1 given as follows.

**[Table 1]**

| <Base sequences of the target DNAs> | | |
|---|---|---|
| Base sequence number 4 | 5'-d (gacaatgtgtacatcaacatcacctaccac) | Perfect matching |
| Base sequence number 5 | 5'-d (gacaatgt**a**tacatcaacatcacctaccac) | 1-base mismatching |
| Base sequence number 6 | 5'-d (gacaa**a**gt**a**tacatcaacatcacctaccac) | 2-base mismatching |
| Base sequence number 7 | 5'-d (gacaatgtgtac**t**tcaacat**gt**cctaccac) | 3-base mismatching |

The melting temperature Tm of the target DNA having a base sequence complementary to that of the probe DNA used in the embodiment was 65 and the temperature of the hybridizations was 55. After hybridization reactions making use of the target DNA solutions listed above were carried out, within a short period of time, the substrate was subjected to a solution replacement process to replace the present solution with a buffer solution having a temperature higher than the melting temperature Tm of the target DNA having a base sequence complementary to that of the probe DNA. In the fifth embodiment, as the replacement solution, a 100 mM HEPES/0.5Tween mixed solution was used and the solution replacement process was carried out for 5 minutes to increase the hybridization temperature to 80.

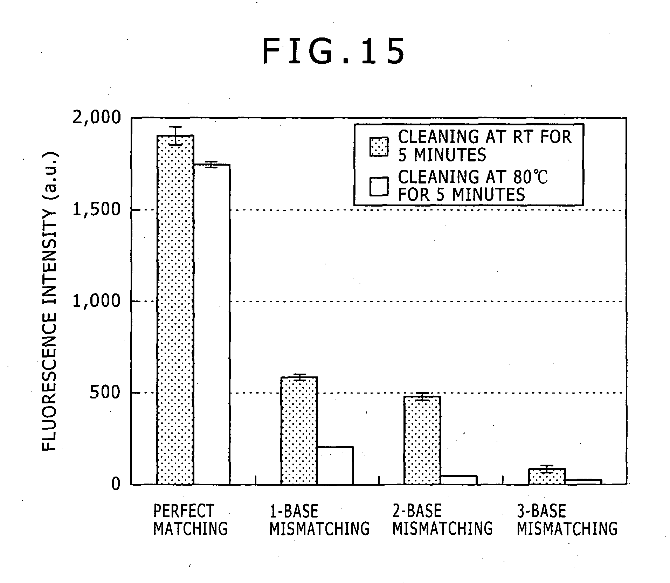

Fig. 15 shows results of measuring the intensity of fluorescence generated by the chromatophore Cy3 above the substrate after carrying out the hybridization reactions and the processes to replace the present solution with a replacement solution having a high temperature. The leftmost dotted and blank bar graphs in the figure represent results of measuring the fluorescence intensity for hybridizations carried out on solution (1) described above before and after the solution replacing process respectively. In this case, the fluorescence intensity represents the hybridization quantity of hybridization carried out on a target DNA having a base sequence complementary to that of the probe DNA. As shown by the bar graphs, the fluorescence intensity measured before the solution replacing process decreases by only a small difference from the fluorescence intensity measured after execution of the solution replacing process. The second dotted and blank bar graphs from the left in the figure represent results of measuring the fluorescence intensity for hybridizations carried out on solution (2) of a target DNA having only one base included in its base sequence as a base not complementary to that of the probe DNA. The third dotted and blank bar graphs from the left in the figure represent results of measuring the fluorescence intensity for hybridizations carried out on solution (3) of a target DNA having only two bases included in its base sequence as bases not complementary to those of the probe DNA. The fourth dotted and blank bar graphs from the left in the figure represent results of measuring the fluorescence intensity for hybridizations carried out on solution (4) of a target DNA having only three bases included in its base sequence as bases not complementary to those of the probe DNA. As is obvious from the second, third and fourth dotted and blank bar graphs from the left, each fluorescence intensity measured before the solution replacing process decreases by a big difference from the fluorescence intensity measured after execution of the solution replacing process. In the case of solution (3) of a 2-base mismatching target DNA having only two bases included in its base sequence as bases not complementary to those of the probe DNA, the hybridization quantity of the 2-base mismatching hybridization can be reduced by at least 90% from the normal hybridization quantity.

As described above, by replacing the reaction space with a solution buffer having a temperature higher than the melting temperature Tm, the mismatching hybridization quantity can be reduced. Thus, it is obvious that the solution replacement process is effective for the improvement of the precision of the process to detect the hybridization reaction.

### Sixth Embodiment

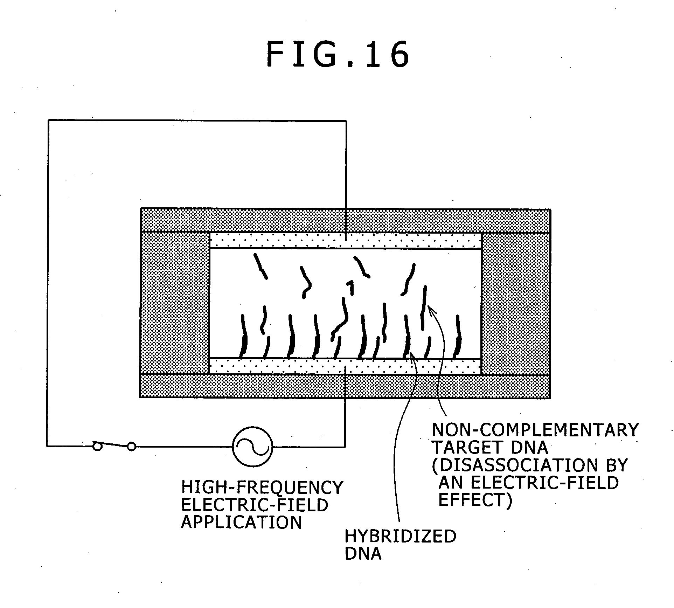

An experiment according to a sixth embodiment was carried out to verify promotion of a process to carry out a disassociation process on a two-strand nucleic acid by applying an electric field to the reaction space. In this embodiment, as shown in Fig. 16, by applying an electric field to the reaction space above a substrate having electrodes after hybridization was advanced in the reaction space, a disassociation process could be carried out only on a selected target DNA obtained as a result of a mis-hybridization.

First of all, a probe DNA was fixed in advance on a substrate having electrodes and, then, each of buffer solutions including target DNAs having base sequence numbers of 4 and 5 corresponding to respectively the perfect matching and the 1-base mismatching, which are shown in Table 1, was introduced into a reaction space above the substrate. The solutions including the target DNAs were each a solvent including 100 mM HEPES and 200 mM NaCl. Subsequently, hybridization reaction was carried out for each of the target-DNA solutions at any arbitrary temperature, which was close to the melting temperature Tm of a DNA having a base sequence complementary to that of the probe DNA or lower than the melting temperature Tm.

After the hybridization was carried out, a 100 mM HEPES/0.5% Tween mixed solution was used for a cleaning process, which was carried out to clean the reaction space at an RT (room temperature) and lasted 5 minutes in order to remove an excessive target DNA from the reaction space. Then, only a buffer solution including 100 mM HEPES and 200 mM NaCl was introduced into the reaction space above the substrate and an electric field was applied between the electrodes. Later on, a 100 mM HEPES/0.5% Tween mixed solution was used for a cleaning process, which was carried out to clean the reaction space at an RT and lasted 5 minutes in order to remove a disassociated excessive target DNA from the reaction space.

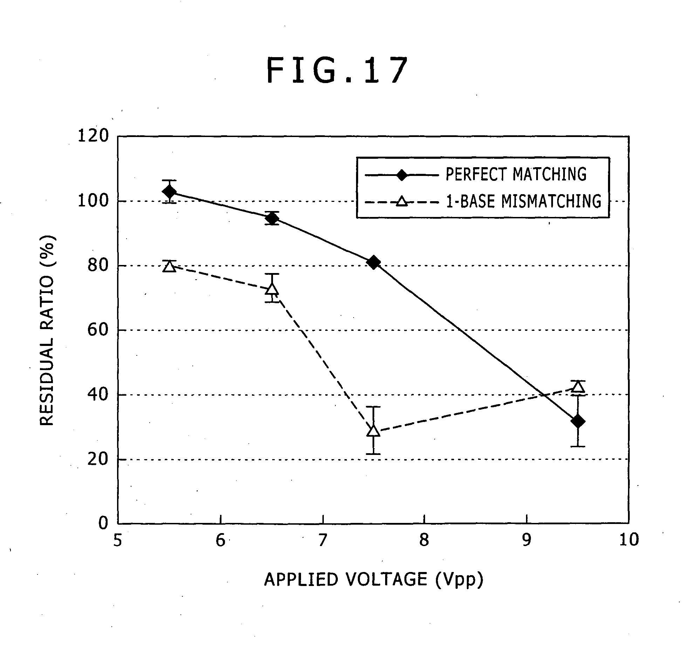

From results of measuring the intensity of fluorescence generated by the chromatophore Cy3 before and after the electric field was applied between the electrodes, residual ratios were found for fluorescence originated by a normal hybridization of a target DNA having a base sequence number of 4 and fluorescence originated by a mis-hybridization of a target DNA having a base sequence number of 5. Fluorescence originated by a normal hybridization is fluorescence generated by a complementary target DNA, which is the target DNA having a base sequence number of 4. On the other hand, fluorescence originated by a mis-hybridization is fluorescence generated by a 1-base mismatching target DNA, which is the target DNA having a base sequence number of 5. The residual ratios are shown in Fig. 17.

As shown in Fig. 17, if an electric field having a frequency of 10 MHz and an amplitude of 5.5 Vpp is applied between the electrodes, the residual ratio for the fluorescence originated by a perfectly matching target DNA does not decrease from 100% but the residual ratio for the fluorescence originated by a 1-base mismatching target DNA decreases from 100% to about 80%. It is thus obvious from these results that the application of the electric field reduces only the residual ratio for the fluorescence originated by the 1-base mismatching target DNA. In the case of applied electric fields each having a voltage amplitude greater than 5.5 Vpp, the residual ratio for the fluorescence originated by the perfectly matching target DNA starts to decrease from 100%, but the residual ratio for the fluorescence originated by the 1-base mismatching target DNA decreases from 100% even more than the residual ratio for the fluorescence originated by the perfectly matching target DNA does. In the case of an applied electric field having a voltage amplitude of 9.5 Vpp, however, the probe DNA fixed on the substrate was damaged so that the hybridization itself was not carried out anymore. That is to say, the voltage amplitude of 9.5 Vpp is not suitable amplitude of the electric field applied between the electrodes.

As described above, by applying an electric field between the electrodes, a disassociation process can be carried out selectively on a selected target DNA obtained as a result of a mis-hybridization. In addition, by controlling the amplitude of the applied electric field, the disassociation process can be carried out more selectively. As a result, the hybridization can be carried out with a higher degree of precision.

It is to be noted that the electrodes are made of a metal such as the aluminum or the gold, or a transparent conductor such as an ITO (Indium Tin Oxide). In some cases, an insulator not shown in the figure may be used for veiling each of the electrodes in order to prevent an electro-chemical reaction of an ion solution from taking place in the reaction space. The insulator is made of a material, which can be SiO₂, SiC, SiN, SiOC, SiOF, TiO₂ or the like. In addition, the electrode structure is by no means limited to the configuration shown in Fig. 16. For example, the electrode structure can be a structure with varying areas of the electrodes facing each other or a structure in which the electrodes facing each other are placed on the same planar surface. In addition, it is desirable to apply an electric field having an AC voltage in order to effectively prevent an electrical decomposition of the ion solution from occurring on the surfaces of the electrodes. In the embodiment, an AC voltage having a frequency in the range 1 kHz to 500 MHz was used as the Voltage of the electric field.

As a principle of the disassociation process for a mis-hybridization, it is possible to make use of a temperature increasing effect exhibited by a buffer solution as a result of applying an electric field, an effect caused by a convection induced in a buffer solution as an effect of applying an external force to a DNA to be disassociated or an effect (or a polarization) exhibited by a direct electric field on a DNA to be disassociated as an effect of applying an external force to the DNA. In order to make the disassociation process easier to carry out for a mis-hybridization with a small number of hydrogen junctions, the disassociation process can be conceivably carried out for a mis-hybridization under any arbitrary voltage applying condition.

In addition, in this embodiment, it is desirable to perform a cleaning process right after the disassociation process carried out for a mis-hybridization by applying an electric field between the electrodes. To put it in more detail, it is preferable to perform a cleaning process while the disassociation process is being carried out for a mis-hybridization or always replace the cleaning solution with a new one.

This is because, even if the selective disassociation process has been carried out successfully for a mis-hybridization, a restoration reaction occurs very fast so that a mis-hybridization can undesirably take place again unless the disassociated DNA is removed away from the reaction space to a location far way from the surface of the substrate. As an alternative, the electric-field application process and the cleaning process are carried out repeatedly in the following order: electric-field application process → cleaning process → electric-field application process → cleaning process and so on. In this way, the number of mis-hybridizations can also be gradually reduced as well.

### Seventh Embodiment

Experiments were carried out in accordance with a seventh embodiment in order to verify that, by combining the method of replacing the present solution with a high-temperature solution in accordance with the fifth embodiment and the method of applying an electric field to the reaction space in accordance with the sixth embodiment, hybridization can be carried out with a higher degree of precision.

To begin with, after hybridization was carried out in the reaction space above the electrode substrate shown in Fig. 16, a process of replacing the present solution with a high-temperature solution and a process of applying an electric field to the reaction space were performed. First of all, each of buffer solutions including the target DNAs shown in Table 1 was introduced into the reaction space above the electrode substrate, on which a probe DNA had been fixed in advance. The solutions including the target DNAs were each a solvent including 100 mM HEPES and 200 mM NaCl. Subsequently, hybridization reaction was carried out for each of the target-DNA solutions at any arbitrary temperature, which was close to the melting temperature Tm of a DNA having a base sequence complementary to that of the probe DNA or lower than the melting temperature Tm. After the hybridization was carried out, a 100 mM HEPES/0.5% Tween mixed solution was used for a cleaning process, which was carried out to clean the reaction space at a room temperature and lasted 5 minutes in order to remove an excessive DNA from the reaction space. Then, as the process of replacing the present solution with a high-temperature solution, the electrode substrate was cleaned by making use of the 100 mM HEPES/0.5% Tween mixed solution at a temperature of 80 for 5 minutes.

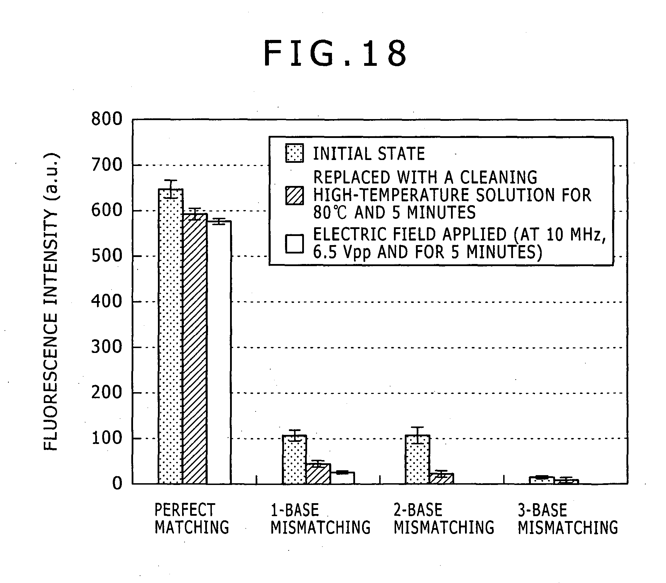

Subsequently, only the solvent including 100 mM HEPES and 200 m MNaCl was introduced into the reaction space above the substrate to serve as a buffer solution, and an electric field having a frequency of 10 MHz and an amplitude of 7.5 Vpp was applied between the electrodes. At that time, in order to remove a DNA, which was set in a free state in the reaction space as a result of a disassociation process, from the reaction space, the electrode substrate was again cleaned by making use of the 100 mM HEPES/0.5% Tween mixed solution at the RT (room temperature) for 5 minutes. After the hybridization (or, strictly speaking, after the re-cleaning process described above) was performed, a high-temperature cleaning process was carried out. After high-temperature cleaning process was carried out, the intensity of fluorescence generated by the chromatophore Cy3 was measured before and after the electric field was applied between the electrodes. Results of the measurement are shown in Fig. 18.

Fig. 18 shows intensities measured as intensities of fluorescence generated by the chromatophore Cy3 in an initial state, in a state after execution of a high-temperature cleaning process and in a state after execution of an electric-field application process for different cases of mismatching. As shown in the figure, fluorescence intensities measured for different cases of mismatching are extremely small in comparison with fluorescence intensities measured for the perfect matching case. In the case of a 1-base mismatching target DNA involved in a mis-hybridization, effects of the process carried out in a short period of time to replace the present solution with a solution having a high temperature and the process carried out to apply an electric field to the reaction space eventually reduce the fluorescence intensity obtained as a measurement result for the mis-hybridization by more than 90%. It is thus obvious that a combination of the technologies according to the fifth and sixth embodiments is effective for the improvement of the precision of hybridization.

From the above description, in the case of the seventh embodiment, the process carried out in a short period of time to replace the present solution with a solution having a high temperature and the process carried out to apply an electric field to the reaction space followed the execution of hybridization in the order explained above. However, the process carried out in a short period of time to replace the present solution with a solution having a high temperature and the process carried out to apply an electric field to the reaction space do not have to follow the execution of hybridization in the order explained above. That is to say, even if the process carried out to apply an electric field to the reaction space precedes the process carried out in a short period of time to replace the present solution with a solution having a high temperature, the same effects can be conceivably reaped. In addition, by repeatedly carrying out the electric-field application process and the cleaning process in the following order: electric-field application process → cleaning process → electric-field application process → cleaning process and so on in the same way as the sixth embodiment, the effect of the reduction of the fluorescence intensities caused by a mis-hybridization can be improved.

### [Industrial Applicability]

The present invention can be applied to a technology for detecting hybridization. For example, the present invention can be applied to a technology for increasing an S/N ratio seen in a process to detect hybridization between a probe nucleic-acid chain and a target nucleic-acid chain.

## Claims

1. A hybridization detection method for increasing an S/N ratio seen at hybridization detection time by carrying out a temperature increasing process of raising the temperature of a reaction space, in which hybridization between a probe nucleic-acid chain and a target nucleic-acid chain is to advance, to a temperature higher than a temperature determined in advance after letting said hybridization advance.

2. The hybridization detection method according to claim 1, whereby after letting said hybridization advance, said temperature increasing process is carried out by raising the temperature of said reaction space to a temperature higher than the melting temperature Tm of a mismatching dual-strand nucleic acid obtained as a result of a mis-hybridization but lower than the melting temperature Tm of a fully matching dual-strand nucleic acid in order to selectively execute a disassociation process on said mismatching dual-strand nucleic acid.

3. The hybridization detection method according to claim 2, wherein
said probe nucleic-acid chain is fixed in said reaction space in a state of having a sequence varying from spot to spot in said reaction space; and
said temperature increasing processes each oriented for one of said spots are carried out independently of each other on the basis of the melting temperatures Tm of said probe nucleic-acid chain and said target nucleic-acid chain.

4. The hybridization detection method according to claim 1, whereby the difference between a reaction equilibrium constant of said hybridization and a reaction equilibrium constant of said mis-hybridization is raised in order to carry out more selectively said disassociation process on said mismatching dual-strand nucleic acid obtained as a result of said mis-hybridization.

5. The hybridization detection method according to claim 1, whereby the temperature of said reaction space is raised in an instant to said temperature determined in advance.

6. The hybridization detection method according to claim 5, whereby the temperature of said reaction space is raised in an instant to said temperature determined in advance by carrying out a high-temperature solution replacement process.

7. The hybridization detection method according to claim 1, whereby said hybridization is detected by making use of an intercalater absorbed by a dual-strand nucleic acid or bound to said dual-strand nucleic acid.

8. The hybridization detection method according to claim 1, whereby said temperature increasing process is carried out in order to selectively execute said disassociation process on said mismatching dual-strand nucleic acid in an attempt to reduce noise fluorescence originated from said mismatching dual-strand nucleic acid.

9. The hybridization detection method according to claim 7, said hybridization detection method at least comprising:
a nucleic acid supplying process of supplying a target nucleic-acid chain to a reaction space on which a probe nucleic-acid chain is maintained or fixed;
a first temperature increasing process of increasing the temperature of said reaction space;
an intercalater supplying process of supplying an intercalater to said reaction space;
a second temperature increasing process of re-increasing the temperature of said reaction space; and
a hybridization detection process of detecting hybridization by making use of said intercalater.

10. The hybridization detection method according to claim 9, whereby said second temperature increasing process is carried out by executing one or both of the following processes:
(1): a process of disassociating an intercalater absorbed by a single-strand nucleic acid existing in said reaction space from said single-strand nucleic acid; and
(2): a process of decomposing a self-loop structure created in a single-strand nucleic acid existing in said reaction space.

11. The hybridization detection method according to claim 1, whereby an electric field is applied to said reaction space at a stage of advancing said hybridization.

12. The hybridization detection method according to claim 1, whereby an electric field is applied to said reaction space while said temperature increasing process is being carried out or after said temperature increasing process has been carried out.

13. The hybridization detection method according to claim 1, whereby
said probe nucleic-acid chain is fixed on said reaction space; and
after said temperature increasing process has been carried out, said reaction space is subjected to a solution cleaning process that leaves a fully matching dual-strand nucleic acid obtained as a result of said hybridization with said probe nucleic-acid chain in said reaction space.

14. The hybridization detection method according to claim 13, whereby said solution cleaning process is carried out after an electric-field applying process of applying an electric field to said reaction space is executed after execution of said temperature increasing process.

15. The hybridization detection method according to claim 14, whereby a process comprising said electric-field applying process and said solution cleaning process is carried out repeatedly.
